(19) Europäisches Patentamt
European Patent Office
Office européen des brevets

(11) **EP 2 807 264 B1**

(12) **EUROPEAN PATENT SPECIFICATION**

(45) Date of publication and mention
of the grant of the patent:
**22.11.2017 Bulletin 2017/47**

(51) Int Cl.:
*C12P 19/00* (2006.01)      *C12N 9/38* (2006.01)
*C12P 19/14* (2006.01)

(21) Application number: **13703536.6**

(22) Date of filing: **25.01.2013**

(86) International application number:
**PCT/EP2013/051477**

(87) International publication number:
**WO 2013/110778 (01.08.2013 Gazette 2013/31)**

(54) **METHOD OF PRODUCING A COMPOSITION CONTAINING GALACTO-OLIGOSACCHARIDES**

VERFAHREN ZUR HERSTELLUNG EINER GALACTO-OLIGOSACCHARID-HALTIGEN ZUSAMMENSETZUNG

PROCÉDÉ DE PRODUCTION DE COMPOSITION CONTENANT DES GALACTO-OLIGOSACCHARIDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **25.01.2012 EP 12152502**
**25.01.2012 US 201261590503 P**

(43) Date of publication of application:
**03.12.2014 Bulletin 2014/49**

(73) Proprietor: **Arla Foods Amba**
**8260 Viby J (DK)**

(72) Inventors:
• **BERTELSEN, Hans**
**DK-6920 Videbæk (DK)**

• **WEJSE, Peter Langborg**
**DK-8200 Aarhus N (DK)**
• **BUSCH, Jon Weis**
**DK-7500 Holstebro (DK)**

(74) Representative: **Guardian**
**IP Consulting I/S**
**Diplomvej, Building 381**
**2800 Kgs. Lyngby (DK)**

(56) References cited:
**EP-A1- 2 138 586      WO-A1-2012/010597**
**WO-A2-01/90317      WO-A2-2009/113030**

• **Madsen, S. et al.: "Bifidobacterium bifidum gene for beta-galactosidase (5509 bp)", , 26 October 2000 (2000-10-26), Retrieved from the Internet: URL:https://www.ncbi.nlm.nih.gov/nuccore/11044948 [retrieved on 2015-04-13]**

**Description**

**FIELD OF THE INVENTION**

The present invention relates to a galacto-oligosaccharide-containing composition as well as an efficient method of producing it.

**BACKGROUND**

Human breast milk is known to contain a number of different oligosaccharides which are ascribed some of the beneficial health effects of breast feeding infants (Kunz *et al.* (2000)). For example, some oligosaccharides, such as FOS, GOS or inulin, are so-called prebiotics, which means that they promote the beneficial bacteria of the gastrointestinal system and disfavour the harmful bacteria. Oligosaccharides are, due to their health promoting effects, frequently used in functional food products, such as infant formulas and clinical nutrition.

There are several approaches to the production of oligosaccharides. One approach is based on isolating oligosaccharides from naturally occurring sources. Fructose-oligosaccharide (FOS) and inulin are for example found naturally in Jerusalem artichoke, burdock, chicory, leeks, onions and asparagus and may be isolated from these crops. Preparation of inulin from chicory roots is e.g. described in Frank (2002). This approach to the production of oligosaccharides is limited by the availability of suitable crops and may be impossible to implement for more complex oligosaccharides.

Another approach is based on enzymatic synthesis in which enzymes catalyse the synthesis of the oligosaccharides. Yun (1996) describes the enzymatic production of fructo-oligosaccharides using enzymes having fructosyltransferase activity and using sucrose as substrate for the enzyme.

Yet an example of enzymatic synthesis is described in WO 01/90,317 A2 which discloses a method of producing galacto-oligosaccharides (GOS) of the formula Gal-Gal-Glc using a special beta-galactosidase enzyme and lactose as substrate. GenBank, accession number AJ224435.2, discloses an updated version (from 5 August 2003) of the sequence of the beta-galactosidase enzyme disclosed in WO 01/90,317.

EP 2 138 586 A1 discloses process for the regio-selective manufacture of disaccharides or oligosaccharides by transglycosylation or hydrolysis of a carbohydrate donator molecule and a glycosyl acceptor molecule in the presence of a glycosidase in the presence of at least one dialkyl amide. However, EP 2 138 586 A1 does not disclose removal, during the incubation, of free leaving groups released from the glycosyl donor.

WO 2009/113,030 A2 describes a process for the production of pure lactose-based galactooligosaccharides using microbial whole cells in a reactor with cross flow hollow fiber micro filtration system. However, WO 2009/113,030 A2 does not contain any teaching regarding the production of hetero-galacto-oligosaccharides using a galactosyl acceptor which is not related to the donor.

WO 2012/010,597 A1 discloses a method of producing compositions containing galacto-oligosaccharides as well as galacto-oligosaccharide-containing compositions as such. However, WO 2012/010,597 A1 does not disclose the removal, during the incubation, of free leaving groups released from the galactosyl donor.

**SUMMARY OF THE INVENTION**

An object of the present invention is to provide improved methods of producing galacto-oligosaccharides, and particularly galacto-oligosaccharides having a different reducing end than the galactosyl donor used during the production.

The present inventors have found that, surprisingly, leaving groups released from the donor during synthesis of galacto-oligosaccharides act as competing galactosyl acceptors and reduces the yield of the above-mentioned galacto-oligosaccharides. This is particularly surprising as initial trials performed by the inventors have indicated that leaving groups, and particularly glucose, are poor galactosyl acceptors. The present inventors have furthermore discovered that the yield of the above-mentioned galacto-oligosaccharides (having a different reducing end than the galactosyl donor) may be increased by removing released leaving groups from the reaction mixture during the incubation of galactosyl donor, galactosyl acceptor and beta-galactosidase enzyme.

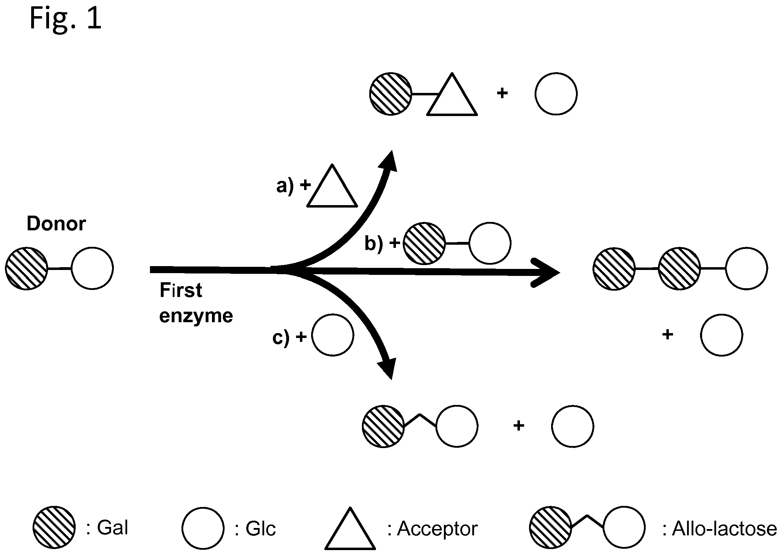

Figures 1, 2 and 3 explain this in further detail.

Figure 1 is a schematic depiction of the main types of reactions that takes place during transgalactosylation. Reaction a) is the desired reaction and involves the transferral of a galactosyl group from the donor (lactose in this example) to the acceptor (e.g. fucose). The products of this reaction is a free leaving group (glucose in this example) and a small oligosaccharide (e.g. Gal-Fuc) having a different reducing end than the galactosyl donor.

Reaction b) is an undesired side reaction which leads to so-called self-galactosylation of the donor, e.g. further galactosylated lactose if the donor is lactose. This by-product is difficult and expensive to remove from the oligosaccharide product. The present inventors have discovered that the level of self-galactosylation can be reduced significantly by using a first enzyme having a high transgalactosylation efficiency (a low T-value) in combination with a relatively low concentration of galactosyl donor.

Reaction c) of Figure 1 is another undesired side reaction which the present inventors have recently discovered. The present inventors have observed surprisingly high levels of allo-lactose in galacto-oligosaccharide compositions and have come to the conclusion that free leaving groups, and particularly, glucose also act as acceptor if its concentration is sufficiently high.

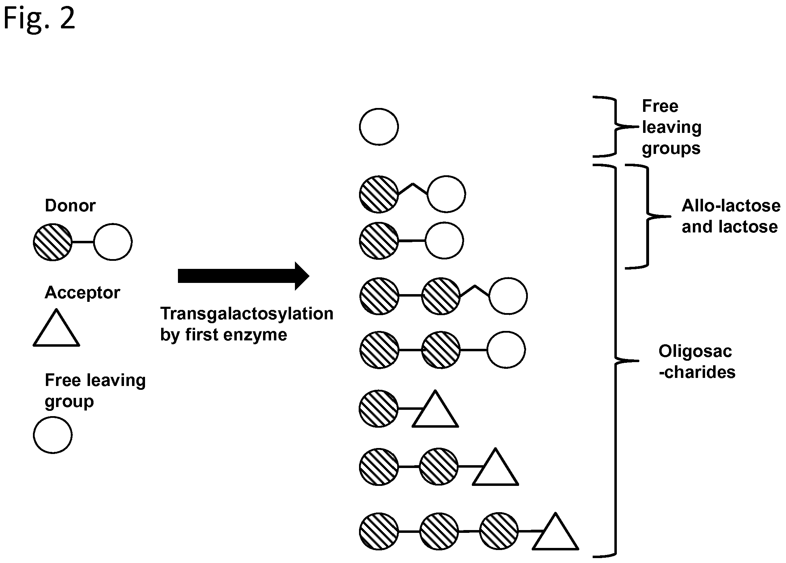

Figure 2 is a schematic illustration of some of the molecular species present in the galacto-oligosaccharide compositions when the free leaving groups are not removed during incubation. In addition to the desired oligosaccharide products derived from galactosyl acceptor, the composition furthermore contains undesired oligosaccharides derived from galactosylation of free leaving groups, e.g. allo-lactose and further galactosylated allo-lactose.

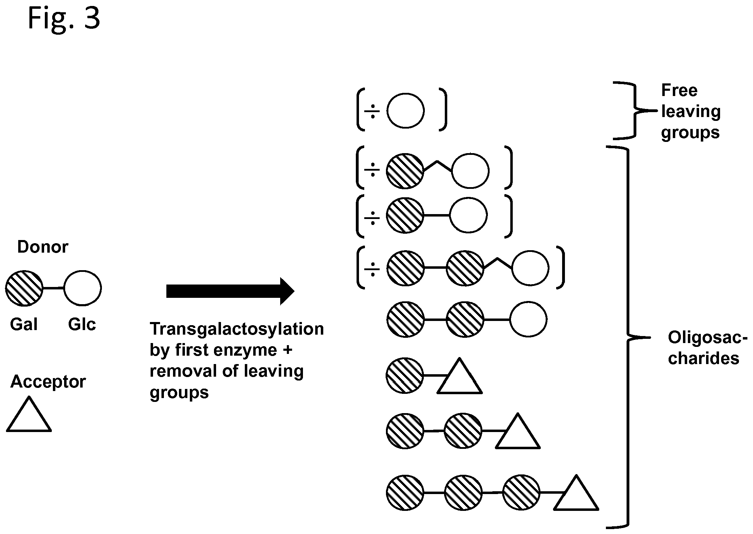

Figure 3 is a schematic illustration of some of the molecular species present in the galacto-oligosaccharide compositions when the free leaving groups are removed during incubation. Contrary to the galacto-oligosaccharide composition illustrated in Figure 2, the present galacto-oligosaccharide composition lacks (or has at least a significantly lower concentration of) free leaving groups, allo-lactose and oligosaccharides derived from allo-lactose.

Thus, an aspect of the invention relates to a method of producing a composition comprising one or more galacto-oligosaccharide(s), the method comprising the steps of:

a) providing a mixture comprising

- a galactosyl donor comprising a galactosyl group bound to a leaving group, which galactosyl donor has a molar weight of at most 350 g/mol,
- a galactosyl acceptor which is different from the galactosyl donor,
  said galactosyl acceptor is a saccharide or a sugar-alcohol, and

wherein the molar ratio between the galactosyl acceptor and the galactosyl donor is at least 1:10, and wherein the mixture comprises at least 0.05 mol/L of the galactosyl acceptor,

b) providing a first enzyme, said first enzyme having beta-galactosidase activity, said first enzyme contacting the mixture, and

c) incubating the mixture and the first enzyme, thereby allowing the first enzyme to release the leaving group of the galactosyl donor and transfer the galactosyl group of the galactosyl donor to the galactosyl acceptor, thus forming the galacto-oligosaccharide, step c) furthermore comprising removing from the incubating mixture a leaving group released from the galactosyl donor and wherein the removal of the leaving group released from the galactosyl donor involves physical removal of free leaving groups from the incubating mixture and/or conversion of free leaving groups into one or more other chemical species which may still be present in the incubating mixture, thereby obtaining the composition comprising the one or more galacto-oligosaccharide(s).

The term "removing a leaving group released from the galactosyl donor" should be understood as physical removal of free leaving groups from the incubating mixture and/or conversion of free leaving groups into one or more other chemical species which may still be present in the incubating mixture. It is preferred that such chemical species do not act as galactosyl acceptors, or at least that they are a less efficient galactosyl acceptor than the free leaving group.

This invention opens up for cheap and efficient production of complex galacto-oligosaccharide compositions in high yield. The present invention furthermore appears to reduce the galactosylation of free leaving groups released from of the galactosyl donor and well as the self-galactosylation of the galactosyl donor, which both result in undesired by-products which are expensive to remove from the composition.

Preferably, the first enzyme has transgalactosylating activity in addition to beta-galactosidase activity. It may also be preferred that the first enzyme has a T-value of at most 0.9.

In the context of the present invention the term "transgalactosylating activity" of a beta-galactosidase enzyme relates to the ability of the enzyme to transfer a galactosyl group from a donor molecule, e.g. a lactose molecule, to a non-water molecule, e.g. another lactose molecule.

The T-value is a measure of the transgalactosylating efficiency of a beta-galactosidase enzyme using lactose both as galactosyl donor and acceptor. The determination of the T-value of a beta-galactosidase enzyme is performed according to the assay and the formula described in Example 3. The T-value is calculated using the formula:

$$\text{T-value} = \frac{\text{amount of produced galactose (in mol)}}{\text{amount of used lactose (in mol)}}$$

A lactase enzyme without any transgalactosylating activity will produce one mol galactose for each used mol lactose and would have a T-value of 1. A beta-galactosidase having an extremely high transgalactosylating activity would use nearly all the galactosyl groups from the lactose for transgalactosylating instead of generating galactose, and would consequently have a T-value near 0.

Yet an aspect of the disclosure relates to a composition comprising one or more galacto-oligosaccharide(s), which composition is obtainable by the method as described herein.

Additional objects and advantages of the invention are described below.

## BRIEF DESCRIPTION OF THE FIGURES

Figure 1 shows a schematic illustration of the types of reactions involved in transgalactosylation.

Figure 2 shows a schematic representation of the reaction products obtained when the free leaving groups are not used.

Figure 3 shows a schematic representation of the reaction products obtained when the free leaving groups are not used.

Figure 4 shows a plot of the integrated response of di-, tri-, and tetrasaccharide of galactosylated fucose after 4 hours of incubation - with and without removal of free leaving groups during the incubation. The free leaving groups (glucose) are removed by means of enzymatic conversion. It is seen that the content of galactosylated fucose increases when the free leaving groups are removed during incubation.

Figure 5 shows a plot of the integrated response of di-, tri-, and tetrasaccharide of galactosylated fucose after 5 hours of incubation - with and without removal of free leaving groups during the incubation. Again, it is seen that the content of galactosylated fucose increases when the free leaving groups are removed during incubation.

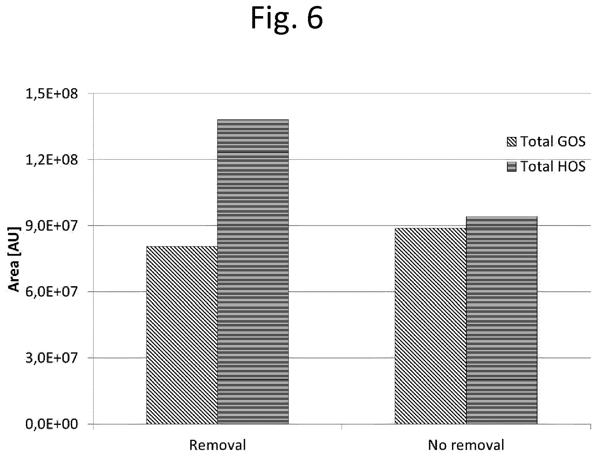

Figure 6 show a plot of the total integrated response of galactosylated fucose compared to the total integrated response of galactosylated donor/leaving group - with and without removal of free leaving groups during the incubation. The total integrated response of galactosylated donor/leaving group is the sum of the integrated responses of Gal-Glc, Gal-Gal-Glc, and Gal-Gal-Gal-Glc molecules. The total integrated response of galactosylated fucose is the sum of the responses of Gal-Fuc, Gal-Gal-Fuc, and Gal-Gal-Gal-Fuc molecules. It is seen that the total integrated response of galactosylated fucose increases significantly when the free leaving groups are removed during incubation while the total integrated response of galactosylated donor/leaving group is almost unchanged.

## DETAILED DESCRIPTION OF THE INVENTION

As mentioned, an aspect of the invention relates to a method of producing a composition comprising one or more galacto-oligosaccharide(s), the method comprising the steps of:

a) providing a mixture comprising

- a galactosyl donor comprising a galactosyl group bound to a leaving group, which galactosyl donor has a molar weight of at most 350 g/mol,
- a galactosyl acceptor which is different from the galactosyl donor,
  said galactosyl acceptor is a saccharide or a sugar-alcohol, and

wherein the molar ratio between the galactosyl acceptor and the galactosyl donor is at least 1:10, and wherein the mixture comprises at least 0.05 mol/L of the galactosyl acceptor,

b) providing a first enzyme, said first enzyme having beta-galactosidase activity, said first enzymes contacting the mixture, and

c) incubating the mixture and the first enzyme, thereby allowing the first enzyme to release the leaving group of the galactosyl donor and transfer the galactosyl group of the galactosyl donor to the galactosyl acceptor, thus forming the galacto-oligosaccharide, step c) furthermore comprising removing from the incubating mixture, a leaving group released from the galactosyl donor and wherein the removal of the leaving group released from the galactosyl donor involves physical removal of free leaving groups from the incubating mixture and/or conversion of free leaving groups into one or more other chemical species which may still be present in the incubating mixture, thereby obtaining the composition comprising the one or more galacto-oligosaccharide(s).

In the context of the present invention, the term "glycosyl group" relates to a group obtained by removing one or two hydroxyl groups from a monosaccharide or a lower oligosaccharide, such as a di- or tri-saccharide, or from corresponding sugar-alcohols. The term is used herein to describe various building blocks of galactosyl donors, galactosyl acceptors and oligosaccharides.

The abbreviations of the most common saccharides and their corresponding glycosyl groups are shown below.

| Saccharide | Abbreviation | Name of glycosyl group |
|---|---|---|
| Glucose | Glc | glucosyl |
| Galactose | Gal | galactosyl |
| fucose | Fuc | fucosyl |
| mannose | Man | mannosyl |
| xylose | Xyl | xylosyl |
| N-acetylgalactosamine | GalNAc | N-acetylgalactosaminyl |
| Lactose | Lac | lactosyl |

In the context of the present invention, the term "oligosaccharide" relates to a molecule comprising at least two glycosyl groups, and preferably at least three, which may be different or the same type. The at least two glycosyl groups are preferably bound via an O-glycosylic bond. An oligosaccharide may be a linear chain of glycosyl groups or it may have a branched structure. Oligosaccharides may e.g. be represented as a stoichiometric formula, e.g. $(Gal)_3Glc$, or as general formulas, e.g. Gal-Gal-Gal-Glc, Gal-Gal-Glc-Gal, or Gal-(Gal-)Glc-Gal. The stoichiometric formulas provide information regarding which glycosyl groups an oligosaccharide, or a group of oligosaccharides, contains, but not the relative position of these, whereas the general formulas also contain general information regarding the relative positions of the glycosyl groups.

In the context of the present invention the term "homo-oligosaccharide" relates to an oligosaccharide containing only one type of glycosyl group. Examples of homo-oligosaccharides are Gal-Gal-Gal-Gal and Glc-Glc-Glc.

In the context of the present invention the term "hetero-oligosaccharide" relates to an oligosaccharide which contains different glycosyl groups, e.g. Gal-Gal-Glc, or Gal-Gal-Fuc.

In the context of the present invention, the prefix "galacto-" used together with the term "oligosaccharide" indicates that the oligosaccharide contains galactosyl groups as the repeating unit. The "homo-" or "hetero-" prefix may be used together with the "galacto-" prefix. Both Gal-Gal-Glc and Gal-Gal-Gal-Gal are galacto-oligosaccharides. Gal-Gal-Glc is a hetero-galacto-oligosaccharide and Gal-Gal-Gal-Gal is a homo-galacto-oligosaccharide.

In the context of the present invention, "X" represents a galactosyl acceptor as defined herein. "-X" represents the glycosyl group corresponding to the galactosyl acceptor, and particularly the glycosyl group bound to another group. "-" symbolises the bond. The glycosyl group is preferably bound via the 3-, 4-, 5- or 6-position of the glycosyl group, and preferably via an O-glycosylic bond.

In the context of the present invention, "Gal-" represents a galactosyl group bound to another group, preferably via the 1-position of the galactosyl group, and preferably via an O-glycosylic bond.

In the context of the present invention, "-Gal-" represents a galactosyl group bound to two other groups. The left bond is preferably made via the 4- or 6-position of the galactosyl group, and preferably via an O-glycosylic bond. The right bond is preferably made via the 1-position of the galactosyl group, and preferably via an O-glycosylic bond.

Bonds between two galactosyl groups are typically 1-4 or 1-6 bonds, and normally O-glycosylic bonds. A bond between a galactosyl group and a nitrogen-containing acceptor may alternatively be an N-glycosylic bond.

Method of the present invention is preferably a method of producing a composition comprising one or more galacto-oligosaccharide(s) using a galactosyl donor and a galactosyl acceptor, which one or more galacto-oligosaccharide(s) have the galactosyl acceptor as its reducing end.

In the context of the present invention the terms "method" and "process" are used interchangeably.

Step a) involves the provision of the mixture in which the oligosaccharides are to be produced.

The mixture is preferably a liquid mixture and may e.g. be an aqueous solution containing the galactosyl acceptor and the galactosyl donor.

In some embodiments of the invention the molar ratio between the galactosyl acceptor and the galactosyl donor of the mixture of step a) is at least 1: 5, preferably at least 1:1, and even more preferably at least 5:1. For example, the molar ratio between the galactosyl acceptor and the galactosyl donor of the mixture of step a) may be at least 10:1, such as at least 15:1.

The molar ratio between the galactosyl acceptor and the galactosyl donor of the mixture of step a) may e.g. be in the range of 1:10-100:1.

In some embodiments of the invention the molar ratio between the galactosyl acceptor and the galactosyl donor of the mixture of step a) is in the range of 1:10-50:1, preferably in the range of 1:5-30:1, and even more preferably in the range of 1:1-20:1. For example, the molar ratio between the galactosyl acceptor and the galactosyl donor of the mixture of step a) may e.g. be in the range of 2:1-40:1, preferably in the range of 4:1-30:1, and even more preferably in the range of 10:1-25:1.

As mentioned, the galactosyl donors contain a galactosyl group covalently bound to a leaving group. The galactosyl group is preferably a $\beta$-D-galactopyranosyl group. Furthermore, the galactosyl group is preferably bound to the leaving group via an O-glycosidic bond from the 1-position of the galactosyl group.

The leaving group of the galactosyl donor may for example be a glycosyl group and/or a sugar-alcohol group. It is particularly preferred that the leaving group of the galactosyl donor is a glucosyl group, i.e. a glucose residue.

If the leaving group is a glycosyl group of a mono- or disaccharide or a corresponding sugar-alcohol, the galactosyl group is preferably bound to the leaving group via an O-glycosidic bond from the 1-position of the galactosyl group, which bond attaches to the 4-position of a monosaccharide-type leaving group or to the 4'-position of a disaccharide-type leaving group.

In the context of the present invention, the phrase "Y and/or X" means "Y" or "X" or "Y and X". Along the same line of logic, the phrase "$X_1$, $X_2$,..., $X_{i-1}$, and/or $X_i$" means "$X_1$" or "$X_2$" or "$X_{i-1}$" or "$X_i$" or any combination of the components : $X_1$, $X_2$,...$X_{i-1}$, and $X_i$.

In some embodiments of the invention the galactosyl donor has a molar weight of at most 1000 g/mol. For example, the galactosyl donor may have a molar weight of at most 500 g/mol. It may even be preferred that the galactosyl donor has a molar weight of at most 350 g/mol.

Disaccharides are a presently preferred type of galactosyl donor. Alternatively, or additionally, tri-saccharides may be used as galactosyl donors as well. Thus, it is envisioned that the mixture may contain a combination of different galactosyl donors.

In some preferred embodiments of the invention the galactosyl donor is lactose. Another example of a useful galactosyl donor is lactulose. Yet an example of a useful galactosyl donor is lactitol.

In the context of the present invention the term "lactose" relates to the disaccharide $\beta$-D-galactopyranosyl-$(1\rightarrow4)$-D-glucose, which is also referred to as milk sugar, and which is the most predominant saccharide of bovine milk.

The galactosyl donor may be provided via any useful galactosyl donor source, both industrially refined sources, such as purified lactose, and/or natural sources, such as whey permeate, i.e. deproteinated whey prepared by ultrafiltration of whey.

The galactosyl acceptor may be any molecule capable of accepting a galactosyl group from the first enzyme and typically contains hydroxyl groups, and preferably alcoholic hydroxyl groups. The term "accepting" means that the galactosyl group of the donor should be covalently bound to the acceptor, e.g. via an O-glycosylic bond.

In some embodiments of the invention the galactosyl acceptor comprises one or more alcoholic hydroxyl group(s). For example, the galactosyl acceptor may be a polyol.

In the context of the present invention the term "polyol" relates to a molecule comprising at least two alcoholic hydroxyl groups.

In some preferred embodiments of the invention the galactosyl acceptor is not lactose. It may furthermore be preferred that the galactosyl acceptor is not glucose.

In some preferred embodiments of the invention the galactosyl acceptor is different from the galactosyl donor. It is particularly preferred to use a relatively cheap galactosyl donor, such as lactose, as galactosyl source, and a biologically interesting acceptor, such as fucose, as galactosyl acceptor.

In some embodiments of the invention the galactosyl acceptor is not lactose, galactose, or glucose.

In some embodiments of the invention the galactosyl acceptor is not glucose or oligosaccharides of the general formula Gal-(Gal)$_i$-Glc, where $i$ is a non-negative integer, i.e. for example 0, 1, 2, 3, or 4.

In some embodiments of the invention the galactosyl acceptor is not galactose or oligosaccharides of the general formula Gal-(Gal)$_i$-Gal, where $i$ is a non-negative integer.

Galactosyl acceptors having various molar weights may be used, but galactosyl acceptors having a molar weight of at least 100 g/mol are presently preferred.

In some embodiments of the invention the galactosyl acceptor has a molar weight of at most 1000 g/mol. For example, the galactosyl acceptor may have a molar weight of at most 500 g/mol. It may even be preferred that the galactosyl acceptor has a molar weight of at most 350 g/mol. The galactosyl acceptor may for example have a molar weight of at most 200 g/mol.

In some preferred embodiments of the invention the galactosyl acceptor is a saccharide. The galactosyl acceptor may for example be a mono-saccharide. Alternatively, the galactosyl acceptor may be a di-saccharide.

For example, the galactosyl acceptor may be a pentose. The galactosyl acceptor may e.g. be arabinose. Another example of a useful pentose is xylose. Yet an example of a useful pentose is ribose. The galactosyl acceptor may for example be a pentose selected from the group consisting of arabinose, xylose, and ribose.

Hexoses are another group of useful galactosyl acceptors. The galactosyl acceptor may e.g. be mannose. Another example of a useful hexose is galactose. Yet an example of a useful hexose is tagatose. A further example of a useful hexose is fructose. The galactosyl acceptor may for example be a hexose selected from the group consisting of mannose, galactose, tagatose, and fructose.

In some preferred embodiments of the invention the galactosyl acceptor is a deoxy-hexose. The galactosyl acceptor may for example be fucose, such as e.g. D-fucose, L-fucose, or a mixture thereof.

Alternatively, the galactosyl acceptor may be an oligosaccharide, such as e.g. a di-saccharide or a tri-saccharide. An example of a useful di-saccharide is maltose. Another example of a useful di-saccharide is lactulose.

Yet a useful group of galactosyl acceptors is saccharide derivatives.

In the context of the present invention the term "saccharide derivative" pertains to a saccharide containing one or more non-hydroxyl functional group(s). Examples of such functional groups are a carboxyl group, an amino group, an N-acetylamino group and/or a thiol group. Saccharides which contain an aldehyde group at the 1-position or a ketone group at the 2-position are not considered saccharide derivatives as such unless the saccharides comprise some of the non-hydroxyl functional groups mentioned above.

An example of a useful saccharide derivative is N-acetyl galactosamine. Another example of a useful saccharide derivative is sialic acid. Yet an example of a useful saccharide derivative is sialyl lactose. Thus, the galactosyl acceptor may be a saccharide derivative selected from the group consisting of N-acetyl galactosamine, sialic acid, and sialyl lactose.

Another group of useful galactosyl acceptors is sugar alcohols. Thus, in some embodiments of the invention the galactosyl acceptor is a sugar alcohol. Examples of useful sugar alcohols are sorbitol, xylitol, lactitol, and/or maltitol.

Contrary to the above-mentioned galactosyl acceptors, the present inventors have found that N-acetyl glucosamine and glucose are less efficient galactosyl acceptors. Thus, in some embodiments of the invention the galactosyl acceptor is not glucose or N-acetyl glucosamine.

The mixture may contain one or more further galactosyl acceptor(s) different from the first type of galactosyl acceptor. The different types of galactosyl acceptors of the mixture may e.g. be selected among the galactosyl acceptor types mentioned herein.

In some preferred embodiments of the invention the produced galactosylated acceptors act as a new type of galactosyl acceptor and can be galactosylated as well. In this way, galacto-oligosaccharides may be produced which have the stoichiometric formula $Gal_{i+1}X$, where $i$ is a non-negative integer. Normally, the most predominant species are $GalX$, $Gal_2X$, and $Gal_3X$.

In other preferred embodiments of the invention the produced galactosylated acceptors act as a new type of galactosyl acceptor and can be galactosylated as well. In this way, galacto-oligosaccharides may be produced which have the general formula $Gal-(Gal)_i-X$, where $i$ is a non-negative integer. Normally, the most predominant species are Gal-X, Gal-Gal-X, and Gal-Gal-Gal-X.

In some embodiments of the invention the mixture of step a) comprises the galactosyl donor in a concentration of at most 0.7 mol/L, preferably at most 0.4 mol/L, and even more preferably at most 0.2 mol/L. The mixture may e.g. comprise the galactosyl donor in a concentration in the range of 0.001-0.7 mol/L, preferably in the range of 0.01-0.5 mol/L, and even more preferred in the range of 0.02-0.2 mol/L.

Alternatively, the mixture of step a) may comprise the galactosyl donor in a concentration of at most 0.3 mol/L, preferably at most 0.1 mol/L, and even more preferably at most 0.05 mol/L. The mixture may e.g. comprise the galactosyl donor in a concentration in the range of 0.001-0.2 mol/L, preferably in the range of 0.005-0.1 mol/L, and even more preferred in the range of 0.01-0.05 mol/L.

It should be noted that galactosylated galactosyl acceptor and galactosylated galactosyl donor may to a limited extent act as a galactosyl donor, but galactosylated galactosyl acceptor and galactosylated galactosyl donor are not considered a galactosyl donor in the context of the present invention and do not contribute to the concentrations or ratios of galactosyl donor mentioned herein. The galactosyl acceptor may be used in a range of difference concentrations. It is, however, preferred to avoid saturating the mixture with the galactosyl acceptor since excess galactosyl acceptor normally has to be removed from the galacto-oligosaccharide-containing composition of the disclosure. In some embodiments of the invention the mixture of step a) comprises the galactosyl acceptor in an amount of at least 0.05 mol/L, preferably at least 0.10 mol/L, and even more preferably at least 0.30 mol/L. Even higher concentrations of the galactosyl acceptor may be preferred, thus the mixture of step a) may e.g. comprise the galactosyl acceptor in an amount of at least 0.5 mol/L, preferably at least 0.7 mol/L, and even more preferably at least 1 mol/L.

The mixture may e.g. comprise the galactosyl acceptor in a concentration in the range of 0.05 mol/L - 5 mol/L, preferably in the range of 0.1 mol/L - 2 mol/L, and even more preferably in the range of 0.3 mol/L - 1 mol/L.

However, in some embodiments a relatively low concentration of the galactosyl acceptor is preferred in which case the mixture may e.g. comprise the galactosyl acceptor in a concentration of at most 2 mol/L, preferably at most 0.5 mol/L, and even more preferably at most 0.2 mol/L. For example, the mixture may comprise the galactosyl acceptor in a concentration in the range of 0.05 mol/L - 2 mol/L, preferably in the range of 0.06 mol/L - 1 mol/L, and even more preferably in the range of 0.08 mol/L - 0.8 mol/L.

In addition to galactosyl acceptor and galactosyl donor, the mixture may furthermore contain various additives for optimizing the conditions for the enzymatic reaction.

The mixture may for example contain one or more pH buffer(s) for adjusting the pH of the mixture to the pH optimum of the first enzyme. Alternatively, or in addition, the mixture may comprise water soluble salts containing one or more metal ions. Depending on the specific first enzyme, metal ions such as $Ca^{2+}$, $Zn^{2+}$, or $Mg^{2+}$ may e.g. be used. Note, however, that some first enzymes are insensitive to the presence of metal ions in the mixture.

Conventional methods of synthesising oligosaccharides often employ water-activity-lowering agents, such as e.g. glycerol, ethylene glycol, propylene glycol, polyethyleneglycol (PEG). The present invention advantageously makes it possible to perform efficient synthesis of galacto-oligosaccharides without the use of such water-activity-lowering agents. Thus, in some preferred embodiments of the invention the mixture contains water-activity-lowering agent in an amount of at most 5% by weight relative to the weight of the mixture, preferably at most 1% by weight, and even more preferably at most 0.1% by weight. For example, the mixture may contain water-activity-lowering agent in an amount of at most 0.05% by weight relative to the weight of the mixture.

The mixture of step a) or the ingredients forming the mixture may have been heat treated before the reaction with first enzyme to avoid microbial growth during the reaction. The usual heat treatment processes, such as pasteurisation (e.g. 72 degrees C for 15 seconds), high pasteurisation (e.g. 90 degrees C for 15 seconds), or UHT treatment (e.g. 140 degrees C for 4 seconds), may be used. Care should be taken when heat treating temperature labile enzymes.

Step b) involves the provision of a first enzyme, which preferably has beta-galactosidase activity. Preferably, the first enzyme has transgalactosylating activity in addition to beta-galactosidase activity. It may also be preferred that the first enzyme has a T-value of at most 0.9. It should be noted that the method may furthermore involve the use of additional enzymes, e.g. enzymes having a different enzymatic activity than beta-galactosidase activity or transgalactosylating activity.

In the context of the present invention the term "beta-galactosidase activity" relates to enzymatic catalysis of the hydrolysis of terminal non-reducing β-D-galactose residues in β-D-galactosides, such as lactose. The first enzyme used in the invention preferably belongs to the class EC 3.2.1.23.

It is preferred that the first enzyme has a T-value of at most 0.9. In some embodiments of the invention, the T-value of the first enzyme is at most 0.8, preferably at most 0.7, and even more preferably at most 0.6. For example, the T-value of the first enzyme may be at most 0.5. Preferably the T-value of the first enzyme may be at most 0.4. It may even be more preferred that the T-value of the first enzyme is at most 0.3.

Even lower T-values may be preferred, such as at most 0.2.

Useful first enzymes may e.g. be derived from a peptide (a β-galactosidase) prepared as described in Examples 1 and 2.

Other examples of useful first enzymes having transgalactosylating activity can be found in WO 2011/120993 A1.

In some embodiments of the invention the first enzyme may contain one or more glycosylated amino acid(s). Alternatively, or in addition, the first enzyme may contain one or more phosphorylated amino acid(s). Alternatively, none of the amino acids of the first enzyme are glycosylated or phosphorylated.

In some preferred embodiments of the invention the first enzyme comprises at least two sub-units, each sub-unit consisting of a first enzyme as defined above.

The first enzyme preferably contacts the mixture and is thereby brought into contact with both the galactosyl acceptor and the galactosyl donor.

In some embodiments of the invention the mixture comprises the first enzyme and/or the second enzyme. The first enzyme and/or the second enzyme may e.g. be present in the mixture in dissolved form, e.g. as single enzyme molecules or as soluble aggregate of enzyme molecules.

In other embodiments of the invention the first enzyme and/or the second enzyme is/are separate from the mixture, but brought in contact with the galactosyl acceptor and the galactosyl donor by contacting the first enzyme and/or the second enzyme with the mixture. For example, first enzyme and/or second enzyme immobilised on a stationary solid phase may be used. Examples of useful stationary solid phases are e.g. a filter, a packed bed of first enzyme-containing particles, or similar structures.

Alternatively, the solid phase may e.g. be a free flowing, particulate solid phase, e.g. organic or inorganic beads, forming part of the mixture.

Details relating to the industrial use of enzymes including immobilisation techniques and suitable solid phase types can be found in Buchholz (2005).

The first enzyme is preferably used in a sufficient activity to obtain an acceptable yield of galacto-oligosaccharides. The optimal activity depends on the specific implementation of the process and can easily be determined by the person skilled in the art.

If a high turn-over of galactosyl donor and a high yield of galacto-oligosaccharide is required, it may be preferred to use the first enzyme in a relatively high activity. For example, the activity of the first enzyme may be such that the turn-over of the galactosyl donor is at least 0.02 mol/(L*h), preferably at least 0.2 mol/(L*h), and even more preferably at least 2 mol/(L*h).

The enzymatic reaction takes place during the incubation of step c). As soon as the mixture is exposed to the right conditions, which may be almost immediately when the galactosyl acceptor and the galactosyl donor are brought into contact with the first enzyme, the transgalactosylation usually starts, and in some embodiments of the invention steps b) and c) occur simultaneously.

The first enzyme is capable of releasing the leaving group of the galactosyl donor and transferring the galactosyl group of the galactosyl donor to the galactosyl acceptor. For example, if the galactosyl donor is lactose, glucose is released and the galactosyl group is transferred to the acceptor. The first enzyme acts as catalyst during the enzymatic reaction.

In some preferred embodiments of the invention the first enzyme furthermore transfers galactosyl groups to already galactosylated galactosyl acceptors, thereby generating galactosyl acceptors containing two, three or even more galactosyl groups.

The pH of the incubating mixture is preferably near the optimum pH of the first enzyme. In some embodiments of the invention the pH of the incubating mixture during step c) is in the range of pH 3-9. For example, the pH of the incubating mixture during step c) may be in the range of pH 4-8, such as in the range of pH 5-7.5.

Similar to the pH, the temperature of the incubating mixture is preferably adjusted to the optimum temperature of the used first enzyme. In some embodiments of the invention the temperature of the incubating mixture of step c) is in the range of 10-80 degrees C. The temperature of the incubating mixture may e.g. be in the range of 20-70 degrees C, preferably in the range of 25-60 degrees C, and even more preferably in the range of 30-50 degrees C.

In the context of the present invention, the term "optimum pH of the first enzyme" relates to the pH where the first enzyme has the highest transgalactosylating activity. Along the same lines, the term "optimum temperature of the first enzyme" relates to the temperature where the first enzyme has the highest transgalactosylating activity.

Step c) may furthermore involve stirring the incubating mixture.

The removal of free leaving groups takes place during the incubation of step c). The removal may for example start immediately when step c) starts or it may be postponed until a significant amount of free leaving groups start building up in the incubating mixture.

The free leaving groups may for example be removed from the incubating mixture by microorganisms present in the incubating mixture.

Thus, in some embodiments of the invention, the method furthermore comprises providing a microorganism which is capable of converting free leaving groups released from the galactosyl donor, and allowing said microorganism, during incubation, to remove a leaving group released from the galactosyl donor.

Useful microorganisms are preferably able to selectively remove the free leaving groups from the incubating mixture and e.g. metabolise or convert these into conversion products that interfere less with the synthesis of galacto-oligosaccharides than the leaving group.

In some preferred embodiments of the invention, the removal rate of the microorganism relative to the free leaving group is at least 10 times higher than its removal rate relative to the galactosyl acceptor.

For example, the removal rate of the microorganism relative to the free leaving group may be at least $10^2$ times higher than its removal rate relative to the galactosyl acceptor, preferably at least $10^3$ times higher, and even more preferred at least $10^4$ times higher than its removal rate relative to the galactosyl acceptor. The removal rate of the microorganism relative to the free leaving group may e.g. be at least $10^5$ times higher than its removal rate relative to the galactosyl acceptor. It may even be preferred that the removal rate of the microorganism relative to the free leaving group is at least $10^6$ times higher, and even more preferred at least $10^7$ times higher than its removal rate relative to the galactosyl acceptor.

In the context of the present invention, the term "removal rate" pertains to the number of moles of free leaving group, donor, acceptor or mono-galactosylated acceptor that the microorganism is capable of removing from the incubating mixture per minute.

In some preferred embodiments of the invention, the removal rate of the microorganism relative to the free leaving group is at least 10 times higher than its removal rate relative to the galactosyl donor.

For example, the removal rate of the microorganism relative to the free leaving group may be at least $10^2$ times higher than its removal rate relative to the galactosyl donor, preferably at least $10^3$ times higher, and even more preferred at least $10^4$ times higher than its removal rate relative to the galactosyl donor. The removal rate of the microorganism relative to the free leaving group may e.g. be at least $10^5$ times higher than its removal rate relative to the galactosyl donor. It may even be preferred that the removal rate of the microorganism relative to the free leaving group is at least

$10^6$ times higher, and even more preferred at least $10^7$ times higher than its removal rate relative to the galactosyl donor.

In some preferred embodiments of the invention, the removal rate of the microorganism relative to the free leaving group is at least 10 times higher than its removal rate relative to the mono-galactosylated galactosyl acceptor.

For example, the removal rate of the microorganism relative to the free leaving group may be at least $10^2$ times higher than its removal rate relative to the mono-galactosylated galactosyl acceptor, preferably at least $10^3$ times higher, and even more preferred at least $10^4$ times higher than its removal rate relative to the mono-galactosylated galactosyl acceptor. The removal rate of the microorganism relative to the free leaving group may e.g. be at least $10^5$ times higher than its removal rate relative to the mono-galactosylated galactosyl acceptor. It may even be preferred that the removal rate of the microorganism relative to the free leaving group is at least $10^6$ times higher, and even more preferred at least $10^7$ times higher than its removal rate relative to the mono-galactosylated galactosyl acceptor.

The microorganism is preferably a yeast or a bacterium.

Saccharomyces cerevisiae is an example of a useful yeast, and useful bacteria could for example be Lac Z-negative E.coli strains or other bacteria which can metabolise glucose but not lactose.

Alternatively, or additionally, free leaving groups may be removed from the incubating mixture by means of specific enzymes that convert the leaving groups into other chemical species.

Thus, in some preferred embodiments of the invention, the method furthermore comprises providing a second enzyme which is capable of converting free leaving groups released from the galactosyl donor and allowing said second enzyme, during incubation, to convert a leaving group released from the galactosyl donor.

The term "converting free leaving groups" pertains to the process of converting the free leaving groups into chemical species which preferably are poorer galactosyl acceptors than the free leaving groups as such. The conversion may involve degradation of the free leaving group into several smaller chemical species or it may simply involve a transformation of the free leaving group into a different chemical species.

The present invention may for example pertain to a method of producing a composition comprising one or more galacto-oligosaccharide(s), the method comprising the steps of:

a) providing a mixture comprising

- a galactosyl donor comprising a galactosyl group bound to a leaving group, which galactosyl donor has a molar weight of at most 350 g/mol,
- a galactosyl acceptor which is different from the galactosyl donor,
  said galactosyl acceptor is a saccharide or a sugar-alcohol, and

wherein the molar ratio between the galactosyl acceptor and the galactosyl donor is at least 1:10, and wherein the mixture comprises at least 0.05 mol/L of the galactosyl acceptor,

b) providing a first enzyme and a second enzyme, said first enzyme having beta-galactosidase activity and a T-value of at most 0.9, said second enzyme being capable of converting free leaving groups released from the galactosyl donor, and said first and second enzymes contacting the mixture, and

c) allowing the first enzyme to release the leaving group of the galactosyl donor and transferring the galactosyl group of the galactosyl donor to the galactosyl acceptor, thus forming the galacto-oligosaccharide, and allowing the second enzyme to convert a leaving group released from the galactosyl donor, thereby obtaining the composition comprising the galacto-oligosaccharide.

The second enzyme may for example have hexose oxidase activity, i.e. belonging to the enzyme class EC 1.1.3.5 and having the ability to catalyze the oxidation of beta-D-glucose with $O_2$ to form D-glucono-1,5-lactone and hydrogen peroxide.

It is even more preferred that the second enzyme has glucose oxidase activity, i.e. belonging to the enzyme class EC 1.1.3.4 and having the ability to catalyze the oxidation of beta-D-glucose with $O_2$ to form D-glucono-1,5-lactone and hydrogen peroxide without significant oxidation of glucose-containing disaccharides such as lactose.

In an aqueous, acidic environment, D-glucono-1,5-lactone typically hydrolyses to form gluconic acid.

Alternatively, the second enzymes may have hexokinase activity (EC 2.7.1.1) or glucokinase activity (EC 2.7.1.2).

Other useful second enzymes may e.g. be found in handbooks which are available to the person skilled in the art.

It is preferred that the second enzyme is selective in its conversion of the free leaving groups and only to a limited extend, and preferably not at all, converts the galactosyl donor, the galactosyl acceptor and/or the galacto-oligosaccharides.

Thus, in some preferred embodiments of the invention, the specificity constant of the second enzyme relative to the free leaving group is at least 10 times higher than its specificity constant relative to the galactosyl acceptor.

For example, the specificity constant of the second enzyme relative to the free leaving group may be at least $10^2$ times higher than its specificity constant relative to the galactosyl acceptor, preferably at least $10^3$ times higher, and even more preferred at least $10^4$ times higher than its specificity constant relative to the galactosyl acceptor. The specificity constant of the second enzyme relative to the free leaving group may e.g. be at least $10^5$ times higher than its specificity constant relative to the galactosyl acceptor. It may even be preferred that the specificity constant of the second enzyme relative to the free leaving group is at least $10^6$ times higher, and even more preferred at least $10^7$ times higher than its specificity constant relative to the galactosyl acceptor.

The term "specificity constant" is determined as $k_{cat}/K_m$ and incorporates the rate constants for all steps in the reaction. Because the specificity constant reflects both affinity and catalytic ability, it is useful for comparing different enzymes against each other, or the same enzyme with different substrates. The theoretical maximum for the specificity constant is called the diffusion limit and is about $10^8$ to $10^9$ ($M^{-1}$ $s^{-1}$). At this point every collision of the enzyme with its substrate will result in catalysis, and the rate of product formation is not limited by the reaction rate but by the diffusion rate.

$k_{cat}$ and $K_m$ of the second enzyme is determined at 37 degrees using 50 mM $Na_3PO_4$ buffer adjusted to pH 6.5. The buffer furthermore contains the salts and co-factors which are required for optimal performance of the enzyme. In some preferred embodiments of the invention, the specificity constant of the second enzyme relative to the free leaving group is at least 10 times higher than its specificity constant relative to the galactosyl donor.

For example, the specificity constant of the second enzyme relative to the free leaving group may be at least $10^2$ times higher than its specificity constant relative to the galactosyl donor, preferably at least $10^3$ times higher, and even more preferred at least $10^4$ times higher than its specificity constant relative to the galactosyl donor. The specificity constant of the second enzyme relative to the free leaving group may e.g. be at least $10^5$ times higher than its specificity constant relative to the galactosyl donor. It may even be preferred that the specificity constant of the second enzyme relative to the free leaving group is at least $10^6$ times higher, and even more preferred at least $10^7$ times higher than its specificity constant relative to the galactosyl donor.

In some preferred embodiments of the invention, the specificity constant of the second enzyme relative to the free leaving group is at least 10 times higher than its specificity constant relative to the mono-galactosylated galactosyl acceptor (Gal-X).

For example, the specificity constant of the second enzyme relative to the free leaving group may be at least $10^2$ times higher than its specificity constant relative to the mono-galactosylated galactosyl acceptor, preferably at least $10^3$ times higher, and even more preferred at least $10^4$ times higher than its specificity constant relative to the mono-galactosylated galactosyl acceptor. The specificity constant of the second enzyme relative to the free leaving group may e.g. be at least $10^5$ times higher than its specificity constant relative to the mono-galactosylated galactosyl acceptor. It may even be preferred that the specificity constant of the second enzyme relative to the free leaving group is at least $10^6$ times higher, and even more preferred at least $10^7$ times higher than its specificity constant relative to the mono-galactosylated galactosyl acceptor.

In some preferred embodiments of the invention, the specificity constant of the second enzyme relative to the free leaving group is:

- at least 10 times higher than its specificity constant relative to the galactosyl donor,
- at least 10 times higher than its specificity constant relative to the galactosyl acceptor, and
- at least 10 times higher than its specificity constant relative to the mono-galactosylated galactosyl acceptor.

For example, the specificity constant of the second enzyme relative to the free leaving group may be:

- at least $10^2$ times higher than its specificity constant relative to the galactosyl donor,
- at least $10^2$ times higher than its specificity constant relative to the galactosyl acceptor, and
- at least $10^2$ times higher than its specificity constant relative to the mono-galactosylated galactosyl acceptor.

Preferably, the specificity constant of the second enzyme relative to the free leaving group is:

- at least $10^3$ times higher than its specificity constant relative to the galactosyl donor,
- at least $10^3$ times higher than its specificity constant relative to the galactosyl acceptor, and
- at least $10^3$ times higher than its specificity constant relative to the mono-galactosylated galactosyl acceptor.

Alternatively, the specificity constant of the second enzyme relative to the free leaving group may be:

- at least $10^4$ times higher than its specificity constant relative to the galactosyl donor,
- at least $10^4$ times higher than its specificity constant relative to the galactosyl acceptor, and

- at least $10^4$ times higher than its specificity constant relative to the mono-galactosylated galactosyl acceptor.

The specificity constant of the second enzyme relative to the free leaving group may be:

- at least $10^5$ times higher than its specificity constant relative to the galactosyl donor,
- at least $10^5$ times higher than its specificity constant relative to the galactosyl acceptor, and
- at least $10^5$ times higher than its specificity constant relative to the mono-galactosylated galactosyl acceptor.

It is even more preferred that the specificity constant of the second enzyme relative to the free leaving group is:

- at least $10^6$ times higher than its specificity constant relative to the galactosyl donor,
- at least $10^6$ times higher than its specificity constant relative to the galactosyl acceptor, and
- at least $10^6$ times higher than its specificity constant relative to the mono-galactosylated galactosyl acceptor.

The specificity constant of the second enzyme relative to the free leaving group may for example be:

- at least $10^7$ times higher than its specificity constant relative to the galactosyl donor,
- at least $10^7$ times higher than its specificity constant relative to the galactosyl acceptor, and
- at least $10^7$ times higher than its specificity constant relative to the mono-galactosylated galactosyl acceptor.

It is particularly preferred that the second enzyme has glucose oxidase activity and the leaving group of the galactosyl donor is a glucosyl group in which case the free leaving group is glucose. If the second enzyme has glucose oxidase activity lactose is a preferred galactosyl donor.

Glucose oxidase requires $O_2$ as oxidant and it may be necessary to add extra $O_2$ (g) to the incubating mixture if the normal amount of dissolved $O_2$ in water is insufficient. It is also possible to add co-factors such as FAD (flavin adenine dinucleotide) or NAD (nicotinamide adenine dinucleotide) to the incubating mixture if required.

As mentioned above, $H_2O_2$ is formed when glucose oxidase catalyses the oxidation of glucose to D-glucono-1,5-lactone, and high levels of $H_2O_2$ may be problematic for the process, e.g. due to undesired oxidation of the used enzymes. In some embodiments of the invention, the method furthermore involves providing a peroxidase or a catalase which contacts the incubating mixture and which catalyses the degradation of $H_2O_2$, e.g. to $H_2O$ and $O_2$. Both glucose oxidases and catalases are well-known in the prior art and commercially available. An example of a useful glucose oxidase is Glyzyme BG (Novozymes, Denmark). An example of a useful catalase enzyme is Catazyme 25L (Novozymes, Denmark). Another example is catalase enzyme from *Micrococcus lysodeikticus* (Sigma-Aldrich, Denmark).

The method may furthermore involve contacting the incubating mixture with a lactonase, and preferably a gluconolactonase (EC 3.1.1.17), i.e. an enzyme capable of hydrolysing D-glucono-1,5-lactone into gluconic acid or its corresponding base gluconate.

Thus, in some preferred embodiments of the invention, the incubating mixture is brought in contact with an enzyme having glucose oxidase activity, an enzyme having glucose oxidase activity catalase activity and an enzyme having gluconolactonase activity.

In some preferred embodiments of the invention, the method furthermore comprises providing a removal agent capable of removing at least some of the conversion product obtained by converting the leaving group with the second enzyme, and allowing the removal agent, during the incubation, to remove at least some of the conversion product.

In the context of the present invention the term "removal agent" pertains to a particulate or molecular agent capable of capturing conversion product and removing it from the incubating mixture, e.g. by precipitation.

The removal agent may for example comprise, or even consist of, a salt of a divalent or trivalent metal ion. Examples of useful metal ions are $Ca^{2+}$, $Fe^{2+}$, $Al^{3+}$, or $Zn^{2+}$.

In preferred embodiments of the invention, the removal agent comprises, or even consists of, $CaCO_3$.

Negatively charged conversion products such as e.g. gluconate, the deprotonated form of gluconic acid, may be removed using anion exchange chromatography.

Thus, the removal agent may comprise, or even consist of, an anion exchange material.

In some embodiments of the invention the anion exchange material comprises a solid phase and one or more cationic group(s).

Preferably, at least some of the cationic groups are attached to the outer surface of the solid phase and/or to the surface of pores which are accessible through the surface of the solid phase.

In some embodiments of the invention the solid phase of the anion exchange material comprises one or more components selected from the group consisting of a plurality of particles, a filter, and a membrane.

The solid phase may for example comprise, or even consists of, polysaccharide. Cross-linked polysaccharides are particularly preferred. Examples of useful polysaccharides are cellulose, agarose, and/or dextran. Alternatively, the solid phase may comprise, or even consists of, a non-carbohydrate polymer. Examples of useful non-carbohydrate polymers are methacrylate, polystyrene, and/or styrene-divinylbenzene.

In some preferred embodiments of the invention the cationic groups comprise, or even consists of, amino groups. Tertiary amino groups are particularly preferred and result in quaternary ammonium groups under appropriate pH conditions. Quaternary ammonium groups provide strong anion exchange characteristics to the anion exchange material.

Alternatively, or additionally, the cationic groups may comprise one or more primary or secondary amino groups. A substantial amount of primary or secondary amino groups typically provides the anion exchange material with weak anion exchange characteristics.

More details regarding anion exchange chromatography and its industrial implementation can be found in *Scopes.*

When an anion exchange material is used for capturing the conversion product, the total binding capacity of the anion exchange material may e.g. be at least 30% (mol/mol) relative to the total amount of conversion product produced during the incubation. For example, the total binding capacity of the anion exchange material may be at least 50% (mol/mol) relative to the total amount of conversion product produced during the incubation. Alternatively, the total binding capacity of the anion exchange material may be at least 80% (mol/mol) relative to the total amount of conversion product produced during the incubation.

When employed, the removal agent is preferably present in an amount sufficient to precipitate or capture the theoretical amount of converted leaving group formed during the synthesis process.

Thus, the total binding capacity of the anion exchange material may e.g. be at least 100% (mol/mol) relative to the total amount of conversion product produced during the incubation. For example, the total binding capacity of the anion exchange material may be at least 150% (mol/mol) relative to the total amount of conversion product produced during the incubation. Alternatively, the total binding capacity of the anion exchange material may be at least 200% (mol/mol) relative to the total amount of conversion product produced during the incubation.

The total amount of conversion product produced during the incubation may for example be estimated from the planned process conditions and kinetic data relating to the used enzyme(s).

Alternatively, the expected amount of released leaving groups may be used as guidance for dosing the anion exchange material. Thus, the total binding capacity of the anion exchange material may e.g. be at least 30% (mol/mol) relative to the total amount of leaving groups released during the incubation. For example, the total binding capacity of the anion exchange material may be at least 50% (mol/mol) relative to the total amount of leaving groups released during the incubation. Alternatively, the total binding capacity of the anion exchange material may be at least 80% (mol/mol) relative to the total amount of leaving groups released during the incubation.

The total binding capacity of the anion exchange material may e.g. be at least 100% (mol/mol) relative to the total amount of leaving groups released during the incubation. For example, the total binding capacity of the anion exchange material may be at least 150% (mol/mol) relative to the total amount of leaving groups released during the incubation. Alternatively, the total binding capacity of the anion exchange material may be at least 200% (mol/mol) relative to the total amount of leaving groups released during the incubation.

The inventors have discovered that the present method surprisingly provides a high yield of galacto-oligosaccharides even though a relatively low concentration of the galactosyl donor is used. The relatively low concentration of galactosyl donor additionally reduces the degree of self-galactosylation of the donor, i.e. when the galactosyl group of a first galactosyl donor is transferred to a second galactosyl donor instead of to a galactosyl acceptor.

In some preferred embodiments of the invention, step c) comprises addition of further galactosyl donor to the mixture. This is particularly preferred when a relatively low concentration of the galactosyl donor is used. By adding more galactosyl donor one avoids the galactosyl donor being depleted in the mixture and the concentration of galactosyl donor may be controlled during the enzymatic reaction.

The addition of further galactosyl donor may involve discrete addition(s) of galactosyl donor, e.g. at least once during the enzymatic reaction. Alternatively, or additionally, the addition of further galactosyl donor may be a continuous addition during the enzymatic reaction. The further galactosyl donor is preferably of the same type as used in step a).

Step c) may for example involve measuring the concentration of galactosyl donor during incubation and adding extra galactosyl donor if the concentration is too low.

In some preferred embodiments of the invention, the concentration of galactosyl donor of the mixture during step c) is maintained at a concentration in the range of 0.01-1 mol/L, preferably in the range of 0.01-0.5 mol/L, and preferably in the range of 0.03-0.3 mol/L.

For example, the concentration of galactosyl donor of the mixture during step c) may be maintained at a concentration in the range of 0.02-0.1 mol/L.

Step c) may furthermore comprise addition of further galactosyl acceptor. This makes it possible to control the concentration of galactosyl acceptor of the mixture during step c) and e.g. to keep the galactosyl acceptor concentration substantially constant if this is desired.

In order to produce significant amounts of galacto-oligosaccharides, which contain two or three transferred galactosyl groups, the process should consume more galactosyl donor than galactosyl acceptor. Thereby more of the galactosyl acceptors will become galactosylated two or three times. Thus, in some preferred embodiments of the invention the molar ratio between the consumed galactosyl donor and the consumed galactosyl acceptor is at least 1:1, and preferably at least 5:1, and even more preferably at least 10:1.

Step c) typically ends by inactivating the enzymes e.g. by denaturing the first enzyme by heat treatment or by breaking the contact between the first enzyme and the carbohydrates of the incubated mixture. If the first enzyme an enzyme immobilised to a solid phase, the contact can e.g. be broken by separating the solid phase and the incubated mixture. If the first enzyme has been dissolved in the mixture, the first enzyme can be separated from the incubated mixture by ultrafiltration using a filter which retains the first enzyme but allows for the passage of oligo-saccharides.

Often it is required to enrich and/or purify the galacto-oligosaccharides of the composition and reduce the concentration of the galactosyl acceptor, the galactosyl donor and the released leaving group.

Thus, in some preferred embodiments of the invention the method furthermore comprises the step:

d) enriching the galacto-oligosaccharides of the composition of step c).

In the context of the present invention, the term "enriching the galacto-oligosaccharides" relates to increasing the relative amount of the galacto-oligosaccharides of the composition on a dry weight basis. This is typically done by removing some of the other solids of the composition, e.g. the lower saccharides, and optionally also the first enzyme, if required.

The enrichment of step d) may for example involve chromatographic separation and/or nanofiltration. Details regarding such processes are described in Walstra *et al.* (2006).

In some embodiments of the invention the enrichment involves that at least 50% (w/w on dry weight basis) of the molecules having a molar weight of at most 200 g/mol are removed from the composition of step c). For example, the enrichment may involve that at least 80% (w/w on dry weight basis) of the molecules having a molar weight of at most 200 g/mol are removed from the composition of step c).

In other embodiments of the invention the enrichment involves that at least 50% (w/w on dry weight basis) of the molecules having a molar weight of at most 350 g/mol are removed from the composition of step c). For example, the enrichment may involve that at least 80% (w/w on dry weight basis) of the molecules having a molar weight of at most 350 g/mol are removed from the composition of step c).

As an alternative, or in addition, to the enrichment it may be preferred that step d) comprises one or more processes which increase the concentration of the galacto-oligosaccharides in the composition. Examples of useful concentration steps are e.g. reverse osmosis, evaporation, and/or spray-drying.

Step d) may furthermore involve removing removal agent and/or converted leaving groups bound to the removal agent from the composition of step c). Such removal may e.g. be performed by filtration, sedimentation, or centrifugation.

The galacto-oligosaccharide-containing composition provided by the method may for example be in the form of a dry powder or in the form of a syrup.

The production of a dry powder typically requires one or more process steps, such as concentrating, evaporating, and/or spray-drying. Thus, in some preferred embodiments of the invention step d) furthermore involves concentrating, evaporating, and/or spray-drying the composition in liquid form to obtain the composition in powder form. It is particularly preferred to spray-dry the liquid composition of step d) to obtain a powdered composition. Step d) may for example comprise the enrichment step followed by concentration step, e.g. nanofiltration, reverse osmosis, or evaporation, followed by a spray-drying step. Alternatively, step d) may comprise the concentration step followed by an enrichment step, followed by a spray-drying step. Concentrating the galacto-oligosaccharides of the composition prior to the enrichment may make the subsequent enrichment process more cost-efficient.

Efficient spray-drying may require addition of one or more auxiliary agent(s), such as maltodextrin, milk protein, caseinate, whey protein concentrate, and/or skimmed-milk powder.

The present process may e.g. be implemented as a batch process. The present process may alternatively be implemented as a fed-batch process. The present process may alternatively be implemented as a continuous process.

The present process may furthermore involve recirculation of first enzyme and/or unused galactosyl acceptor back to the mixture. The recirculation may e.g. form part of step d). For example, step d) may involve separating galactosyl acceptor and/or the first enzyme from the galacto-oligosaccharide-containing composition and recirculating galactosyl acceptor and/or first enzyme to step a) or c). In the case of a batch process or a fed-batch process, the galactosyl acceptor and/or the first enzyme may be recirculated to the mixture of the next batch.

In the case of a continuous process, the galactosyl acceptor may be recirculated back to part of the process line corresponding to step a) or step c). The first enzyme may be recirculated back to part of the process line corresponding to step b) or step c).

It should be noted that the details and features related to steps a) and b) need not relate to the actual start of a production process but should at least occur sometime during the process. However, in some embodiments of the invention the concentration of the galactosyl donor is kept within the range described in step a) during the entire duration of step c).

If the method is implemented as a batch or feed batch process, step a) preferably pertains to the composition of the mixture when the synthesis starts. If the method is a continuous process, step a) preferably pertains to the composition of the mixture during the synthesis under steady-state operation.

It may be perceived as desirable that the level of galactosylated galactosyl donor is kept as low as possible, as galactosylated galactosyl donor may be perceived as an undesired impurity, which is tricky to separate from the galactosylated galactosyl acceptor. In some preferred embodiments of the invention, the incubating mixture of step c) contains at most 0.5 mol/L galactosylated galactosyl donor. The incubating mixture of step c) may for example contain at most 0.1 mol/L galactosylated galactosyl donor. Even more preferably the incubating mixture of step c) contains at most 0.01 mol/L galactosylated galactosyl donor, and preferably substantially no galactosylated galactosyl donor.

In some preferred embodiments of the invention where the donor is lactose, the total concentration of allo-lactose and galactosylated allo-lactose is kept as low as possible, as these compounds also are perceived as an undesired impurities, which is difficult to separate from the galactosylated galactosyl acceptor.

In some preferred embodiments of the invention, the incubating mixture of step c) contains a total amount of allo-lactose and galactosylated allo-lactose of at most 0.5 mol/L. The incubating mixture of step c) may for example contain a total amount of allo-lactose and galactosylated allo-lactose of at most 0.1 mol/L. Even more preferably the incubating mixture of step c) contains a total amount of allo-lactose and galactosylated allo-lactose of at most 0.01 mol/L, and preferably substantially no allo-lactose and galactosylated allo-lactose at all.

Yet an aspect of the disclosure relates to a composition comprising galacto-oligosaccharides, which composition is obtainable by the method as defined herein.

A further aspect of the disclosure is a galacto-oligosaccharide-containing composition, e.g. the above-mentioned composition, said galacto-oligosaccharide-containing composition comprising:

- a first galacto-oligosaccharide having the general formula Gal-X,
- a second galacto-oligosaccharide having the stoichiometric formula $(Gal)_2X$, such as e.g. the general formula Gal-Gal-X,
- a third galacto-oligosaccharide having the stoichiometric formula $(Gal)_3X$, such as e.g. the general formula Gal-Gal-Gal-X, and

wherein X is a glycosyl group, which is not lactosyl or glucosyl.

The galacto-oligosaccharide-containing composition described herein may for example be a food ingredient. As described above, "X" or "-X" is preferably a glycosyl group of one of the galactosyl acceptors mentioned herein.

In some embodiments of the invention "X" or "-X" is a glycosyl group of a monosaccharide, which is not glucose. In other embodiments of the invention "-X" is a glycosyl group of a disaccharide, which is not lactose.

In some preferred embodiments of the invention "X" or "-X" is a fucosyl group. In other preferred embodiments of the invention "X" or "-X" is a galactosyl group.

In some preferred embodiments of the disclosure the galacto-oligosaccharide-containing composition has a molar ratio between:

- the total amount of the galacto-oligosaccharides Gal-X, $(Gal)_2X$, and $(Gal)_3X$, and
- the total amount of the galacto-oligosaccharides Gal-Glc, $Gal_2Glc$, $Gal_3Glc$ of at least 5:95. For example, the above-mentioned molar ratio may be at least 1:4, preferably at least 1:1, and even more preferably at least 2:1. It may even be preferred that the above-mentioned molar ratio is at least 5:1, preferably at least 10:1, and even more preferably at least 20:1.

In other preferred embodiments of the disclosure the galacto-oligosaccharide-containing composition has a molar ratio between:

- the total amount of the galacto-oligosaccharides Gal-X, Gal-Gal-X, and Gal-Gal-Gal-X, and
- the total amount of the galacto-oligosaccharides Gal-Glc, Gal-Gal-Glc, Gal-Gal-Gal-Glc

of at least 5:95. For example, the above-mentioned molar ratio may be at least 1:4, preferably at least 1:1, and even more preferably at least 2:1. It may even be preferred that the above-mentioned molar ratio is at least 5:1, preferably at least 10:1, and even more preferably at least 20:1.

It is even possible that the galacto-oligosaccharide-containing composition does not contain any galacto-oligosaccharides of the formula Gal-Glc, Gal-Gal-Glc, and Gal-Gal-Gal-Glc at all.

In some embodiments of the disclosure the galacto-oligosaccharide-containing composition has a molar ratio between the first galacto-oligosaccharide, the second galacto-oligosaccharide, and the third galacto-oligosaccharide in the range of 50-99 : 1-45 : 0.5-25.

In other embodiments of the disclosure the galacto-oligosaccharide-containing composition has a molar ratio between the first galacto-oligosaccharide, the second galacto-oligosaccharide, and the third galacto-oligosaccharide in the range of 20-45 : 20-45: 20-45.

In further embodiments of the disclosure the galacto-oligosaccharide-containing composition has a molar ratio between the first galacto-oligosaccharide, the second galacto-oligosaccharide, and the third galacto-oligosaccharide in the range of 0.5-25 : 1-45 : 50-98.

In some preferred embodiments of the disclosure the galacto-oligosaccharide-containing composition comprises a total amount of the first galacto-oligosaccharide, second galacto-oligosaccharide, and third galacto-oligosaccharide of at least 10% by weight relative to the total weight of the galacto-oligosaccharide-containing composition. For example, the galacto-oligosaccharide-containing composition may comprise a total amount of the first galacto-oligosaccharide, second galacto-oligosaccharide, and third galacto-oligosaccharide of at least 20% by weight relative to the total weight of the galacto-oligosaccharide-containing composition, preferably at least 30% by weight, even more preferably at least 40% relative to the total weight of the galacto-oligosaccharide-containing composition.

Even higher levels of the first, second, and third galacto-oligosaccharides may be preferred. Thus, in some preferred embodiments of the disclosure the galacto-oligosaccharide-containing composition comprises a total amount of the first galacto-oligosaccharide, second galacto-oligosaccharide, and third galacto-oligosaccharide of at least 50% by weight relative to the total weight of the galacto-oligosaccharide-containing composition. For example, the galacto-oligosaccharide-containing composition may comprise a total amount of the first galacto-oligosaccharide, second galacto-oligosaccharide, and third galacto-oligosaccharide of at least 60% by weight relative to the total weight of the galacto-oligosaccharide-containing composition, preferably least 70% by weight, even more preferably at least 80% relative to the total weight of the galacto-oligosaccharide-containing composition.

Yet an aspect of the disclosure relates to a food product comprising the galacto-oligosaccharide-containing composition described herein.

In some embodiments of the disclosure the food product is a functional food product such as infant formula or a product for clinical nutrition.

In other embodiments of the disclosure the food product is a baked product, e.g. comprising baked dough, such as bread or similar products.

In further embodiments of the disclosure the food product is a dairy product, e.g. a fresh dairy product such as milk, or a fermented dairy product such as yoghurt.

In still further embodiments of the disclosure the food product is a pet food product.

It should be noted that embodiments and features described in the context of one of the aspects of the present invention also apply to the other aspects of the invention.

The invention will now be described in further details in the following non-limiting examples.

**EXAMPLES**

Example 1: Preparation of a beta-galactosidase having transgalactosylating activity

A working volume of 750 mL fermentation medium was inoculated with a 2 mL starter-culture of Lysogeny broth (LB) medium with 100 mg/L ampicillin with an $OD_{600}$ of 3.0 grown for 12 hours. The fermentation was performed in EC medium containing 2 % (w/v) yeast extract, 2 % (w/v) soy peptone, 1 % (w/v) glucose and 100 mg/L ampicillin. The *E. coli* strain expressing OLGA347 β-galactosidase (having the sequence Val (33) - Ile (1174) was prepared as described earlier (Jørgensen *et al.,* WO 01/90,317 A2 and US patent No. 6,555,348 B2, Examples 1 and 2). The fermentor was from Applikon with glass dished bottom vessels with a total volume of 2 L and equipped with two Rushton impellers. During the fermentation, pH was maintained at pH 6.5 by appropriate addition of 2 M NaOH and 2 M $H_3PO_4$ and temperature was controlled at 37 degrees C. Oxygen was supplied by bubbling with air at a rate of 1-2 L/min, and $pO_2$ was maintained at 30% by increasing the agitation rate. Growth was followed by off-line $OD_{600}$ readings. The culture was harvested by centrifugation after approximately 10 h of growth at an $OD_{600}$ value of 29.7. The 650 mL culture supernatant was stored at - 20 degrees C. The periplasmic proteins were isolated from the cell pellet by osmotic shock by resuspending the cell pellet in 200 mL sucrose buffer (30 mM Tris-HCl, 40% sucrose, 2 mM EDTA, pH 7.5) and incubating for 10 min at room temperature. After centrifugation, the supernatant was discarded and the pellet resuspended in 200 mL of cold water. 83 $\mu$L of a saturated $MgCl_2$ solution was added, and the supernatant containing the periplasmic proteins were collected by a centrifugation step. The periplasmic fraction was filter sterilized through a 0.2 $\mu$m Millipak 40 filter and stored at -20 degrees C.

The β-galactosidase activity of the 200 mL periplasmic fraction and the 650 mL culture supernatant was determined using *o*-nitrophenyl-*β*-D-galactopyranoside (OPNG) as a substrate according to protocol (J. Sambrook and D.W. Russell, Molecular Cloning - A laboratory manual, 3rd edition (2001), pp. 17.48-17.51). The majority of the activity was found in the periplasmic fraction (525 units, corresponding to 98%).

Example 2: Preparation of a second beta-galactosidase having transgalactosylating activity

A second beta-galactosidase (OLGA917) was prepared along the lines described in Example 1 but based on the expression of the amino acid sequence Val (33) - Glu (917).

Example 3: Determination of the T-value of a beta-galactosidase enzyme

The T-value of a beta-galactosidase enzyme is determined according to the assay and formula given below.

Assay:

Prepare 3.3 mL enzyme solution consisting of the beta-galactosidase enzyme to be tested, 10 mM sodium citrate, 1 mM magnesium citrate, 1 mM calcium-citrate, Milli-Q water (Millipore, USA), and having a pH of 6.5. The enzyme solution should contain the beta-galactosidase enzyme in an amount sufficient to use 33% (w/w) of the added lactose in 1 hour under the present assay condition. The temperature of the enzyme solution should be 37 degrees C.
At time = $T_0$ 82.5 mg lactose monohydrate (for biochemistry, Merck Germany) is added to and mixed with the enzyme solution, and the mixture is subsequently incubated at 37 degrees C for 4 hours. Precisely 1 hour after $T_0$ a 100 $\mu$L sample is collected and is diluted 1:5 with Milli-Q water and inactivated by heating to 85 °C for 10 min. The inactivated mixture is kept at -20 degrees C until the characterization.

Characterisation:

The determination of the amount (in mol) of produced galactose and the amount of used lactose (in mol) may be performed using any suitable analysis technique. For example, the diluted mixture may be analyzed by HPLC according to the method described by Richmond *et al.* (1982) and Simms *et al.* (1994). Other useful analysis techniques are described in El Razzi (2002).
Another example of a suitable analysis technique is ISO 5765-2:2002 (IDF 79-2: 2002) "Dried milk, dried ice-mixes and processed cheese - Determination of lactose content - Part 2: Enzymatic method utilizing the galactose moiety of the lactose".

Calculation of the T-value:

The T-value is calculated according to the following formula using the data obtained from the characterization of the diluted mixture of the assay:

$$\text{T-value} = \frac{\text{amount of produced galactose (in mol)}}{\text{amount of used lactose (in mol)}}$$

Example - T-value of the OLGA347 enzyme:

The above-mentioned assay was performed using the OLGA347 enzyme of Example 1.
The diluted mixture obtained from the assay was analyzed with respect to converted (i.e. used) lactose and generated galactose via analytical HPLC. The HPLC apparatus was from Waters and equipped with a differential refractometer (RI-detector) and a BioRad Aminex HPX-87C column (300x7.8 mm, 125-0055). Elution of saccharides was performed isocratically with 0.05 g/L CaAcetate, a flow rate of 0.3 mL/min. and an injection volume of 20 $\mu$L.
The obtained data was appropriately baseline corrected by automated software, peaks were individually identified and integrated. Quantification was performed by using external standards of lactose monohydrate (for biochemistry, Merck, Germany), D-(+)-glucose monohydrate (for biochemistry, Merck Eurolab, France), and D-(+)-galactose ($\geq$99%, Sigma-Aldrich, Italy).
The conversion of lactose and the formation of galactose to each time T was calculated from the quantified data. At time T=1h 29% of the lactose in the collected 100 $\mu$L sample had been converted, which corresponds to 2.3 $\mu$mol lactose. At time T=1 0.5 $\mu$mol galactose had been formed in the collected 100 $\mu$L sample. The T-value can therefore be calculated to 0.5 $\mu$mol / 2.3 $\mu$mol = 0.2.
The diluted mixture obtained from the assay was also analyzed with respect to converted (i.e. used) lactose and generated galactose via the enzymatic method ISO 5765-2. A Boehringer Mannheim Lactose/D-Galactose test-kit from R-Biopharm (Cat. No. 10 176 303 035) was used and the test performed according to protocol. The enzymatic method confirmed a T-value of the OLGA347-enzyme of 0.2.

Example - T-value of the OLGA917 enzyme:

The above-mentioned T-value assay was performed using the OLGA917 enzyme produced in Example 2 and the T-value was determined following the procedure explained above.
The T-value of the OLGA917 enzyme was determined to 0.3.

Example - T-value of conventional lactase enzyme:

The above-mentioned assay was performed using the commercially available conventional lactase enzyme Lactozym Pure 2600L (Novozymes, Denmark). The diluted mixture obtained from the assay was analyzed as described for the OLGA347 enzyme. Tri- and tetra-saccharides were not present in detectable amounts and equal amounts of glucose and galactose were seen. The corresponding T-value is 1.
The T-values of commercially available beta-galactosidase from *Escherichia coli* (Product number: G6008, Sigma-Aldrich, Germany) and *Aspergillus oryzae* (Product number: G5160, Sigma-Aldrich, Germany) have also been determined, and both enzymes have a T-value of approx. 1.0.

Example 4: Synthesis of L-fucosyl-containing hetero-galacto-oligosaccharides by sequential addition of donor molecules and conversion of glucose leaving groups

L-(-)-Fucose and lactose monohydrate in amounts as found in Table 7 were dissolved in 100 mL MilliQ water. Each sample was maintained at a temperature of 37 degrees C and pH is maintained at 6.5 by continuous addition of NaOH. Air was continuously pumped through the mixture at a rate of 100 L/min. Glucose Oxidase enzyme, GOX, DuPont, Denmark, was added to sample 2. Glucose Oxidase enzyme, Gluzyme, Novozymes, Denmark, was added to sample 3. Catalase enzyme, from *Micrococcus lysodeikticus,* Sigma-Aldrich, Denmark, was added to sample 2 and 3. OLGA917 enzyme of Example 2 was added to each sample. MilliQ water was added to a total process volume of 250 mL. This time was defined as T=0. Lactose monohydrate was added to the samples over a 5 hour period in 30 min. intervals.

Table 7:

| Sample | 1 (Control) | 2 | 3 |
|---|---|---|---|
| **Start concentration of L-Fucose** | 10 % | 10 % | 10 % |
| **Start concentration of lactose monohydrate** | 5 % | 5% | 5% |
| **Addition of lactose / 30 min.** | 2.63 g | 2.63 g | 2.63 g |
| **Amount of Glucose oxidase** | - | 100.45 mg | 110.50 mg |
| **Specific activity of Glucose oxidase** | - | 11,000 U/g | 10,000 U/g |
| **Amount of catalase** | - | 20 $\mu$L | 20 $\mu$L |
| **Specific activity of catalase** | - | 170,000 U/mL | 170,000 U/mL |
| **Amount of OLGA enzyme** | 5 mL | 5 mL | 5 mL |

Test samples from T=4 h and T= 5h were analysed by HPLC according to Example 7 and were found to contain significant amounts of galactosylated fucose.

Example 5: Synthesis of L-fucosyl-containing hetero-galacto-oligosaccharides and conversion and removal of glucose leaving groups but without sequential addition of donor molecules

L-(-)-Fucose and lactose monohydrate in amounts as found in Table 8 were dissolved in 100 mL MilliQ water. Each sample was maintained at a temperature of 37 degrees C and the pH was maintained at 6.5 by continuous addition of NaOH. Air was continuously pumped through the mixture at a rate of 100 L/min. Anion exchange resin, Dowex 66, Sigma-Aldrich, USA was prepared and added to sample 3. Glucose Oxidase enzyme, GOX, DuPont, Denmark, was added to sample 2 and 3. Catalase enzyme, from *Micrococcus lysodeikticus,* Sigma-Aldrich, Denmark, was added to sample 2 and 3. OLGA917 enzyme of Example 2 was added to each sample. MilliQ water was added to a total process volume as found in Table 8. This time was defined as T=0.

Table 8:

| Sample | 1 (Control) | 2 | 3 |
|---|---|---|---|
| Start concentration of L-Fucose | 10 % | 10 % | 10 % |
| Start concentration of lactose monohydrate | 15 % | 15% | 15% |
| Amount of Glucose oxidase | - | 100.45 mg | 100.45 mg |
| Specific activity of Glucose oxidase | - | 11,000 U/g | 11,000 U/g |
| Amount of catalase | - | 20 μL | 20 μL |
| Specific activity of catalase | - | 170,000 U/mL | 170,000 U/mL |
| Volume of ion exchange resin | - | - | 40 mL |
| Weak base capacity | - | - | 1.35 meq/mL |
| Amount of OLGA enzyme | 5 mL | 5 mL | 5 mL |
| Total process volume | 250 mL | 250 mL | 250 mL |

Test samples from T=4 h and T= 5h were analysed by HPLC according to Example 7 and were also found to contain significant amounts of galactosylated fucose.

Example 6: Synthesis of L-fucosyl-containing hetero-galacto-oligosaccharides by sequential addition of donor molecules, conversion of glucose leaving groups, and removal of converted leaving groups

L-(-)-Fucose and lactose monohydrate in amounts as found in Table 9 were dissolved in 100 mL MilliQ water. Each sample was maintained at a temperature of 37 degrees C and the pH was maintained at 6.5 by continuous addition of NaOH. Air was continuously pumped through the mixture at a rate of 100 L/min. Anion exchange resin, Dowex 66, Sigma-Aldrich, USA was prepared and added to each sample. Glucose Oxidase enzyme, GOX, Danisco/DuPont, Denmark, was added to sample 2. Glucose Oxidase enzyme, Gluzyme, Novozymes, Denmark, was added to sample 3. Catalase enzyme, from *Micrococcus lysodeikticus,* Sigma-Aldrich, Denmark, was added to sample 2 and 3. OLGA917 enzyme of Example 2 was added to each sample. MilliQ water was added to a total process volume of 250 mL. This time was defined as T=0. Lactose monohydrate was added to the samples over a 5 hour period in 30 min. intervals.

Table 9:

| Sample | 1 (Control) | 2 | 3 |
|---|---|---|---|
| Start concentration of L-Fucose | 10 % | 10 % | 10 % |
| Start concentration of lactose monohydrate | 5 % | 5% | 5% |
| Addition of lactose / 30 min. | 1,06 g | 1,06 g | 1,06 g |
| Amount of Glucose oxidase | - | 40 mg | 45 mg |
| Specific activity of Glucose oxidase | - | 11,000 U/g | 10,000 U/g |
| Amount of catalase | - | 10 μL | 10 μL |
| Specific activity of catalase | - | 170,000 U/mL | 170,000 U/mL |
| Volume of ion exchange resin | 40 mL | 40 mL | 40 mL |
| Weak base capacity | 1.35 meq/mL | 1.35 meq/mL | 1.35 meq/mL |
| Amount of OLGA enzyme | 2 mL | 2 mL | 2 mL |

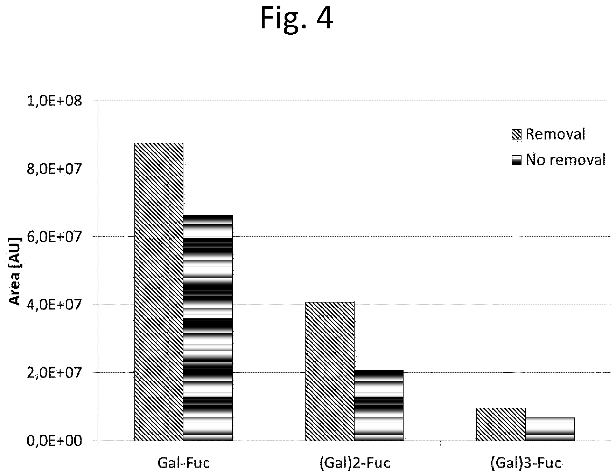

At times T=4 and 5 h 1500 μL test samples were taken and analysed by HPLC and Mass Spectroscopy according to Example 7. The samples were found to contain significant amounts of galactosylated fucose. The results of the Mass Spectroscopy analysis is illustrated in Figures 4-6.

Figure 4 shows a plot of the integrated response of di-, tri-, and tetrasaccharide of galactosylated fucose after 4 hours of incubation - with and without removal of free leaving groups during the incubation. The free leaving groups (glucose) are removed by means of enzymatic conversion. It is seen that the content of galactosylated fucose increases when the free leaving groups are removed during incubation.

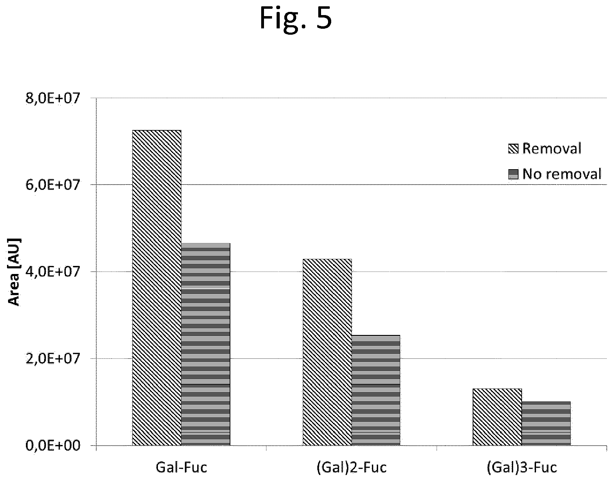

Figure 5 shows a plot of the integrated response of di-, tri-, and tetrasaccharide of galactosylated fucose after 5 hours of incubation - with and without removal of free leaving groups during the incubation. Again, it is seen that the content of galactosylated fucose increases when the free leaving groups are removed during incubation.

Figure 6 show a plot of the total integrated response of galactosylated fucose (total HOS) compared to the total integrated response of galactosylated donor/leaving group (total GOS) - with and without removal of free leaving groups during the incubation. The total integrated response of galactosylated donor/leaving group is the sum of the integrated responses of Gal-Glc, Gal-Gal-Glc, and Gal-Gal-Gal-Glc molecules. The total integrated response of galactosylated fucose is the sum of the responses of Gal-Fuc, Gal-Gal-Fuc, and Gal-Gal-Gal-Fuc molecules. It is seen that the total integrated response of galactosylated fucose increases significantly when the free leaving groups are removed during incubation while the total integrated response of galactosylated donor/leaving group is almost unchanged.

Conclusion:

This example demonstrates that the ratio between the galactosylated acceptor (the intended product) and galactosylated donor/leaving groups (by-product) is increased significantly when the free leaving groups are removed during incubation.

Example 7: Characterisation of samples containing L-fucosyl-containing hetero-galacto-oligosaccharides

Collected samples were diluted 1:10 with Milli-Q water and inactivated by heating to 85 °C for 15 min. The inactivated mixture was kept at -20 degrees C until the characterization.

Samples were characterized by use of analytical HPLC. The samples were filtered using a 0.22 $\mu$m filter. The HPLC apparatus was from Waters and equipped with a differential refractometer (RI-detector) and a BioRad Aminex HPX-87C column (300x7.8 mm, 125-0055). Elution of saccharides was performed isocratically with 0.05 g/L CaAcetate, a flow rate of 0.3 mL/min. and an injection volume of 20 $\mu$L.

Mass spectrometry analysis was performed with an Agilent 1200 API-ES LC/MSD Quadropole 6410 scanning masses between 100 and 1000 amu (gas temperature: 350 °C, drying gas flow: 13.0 L/min, nebulizer pressure: 40 psig). The column used for LC separation is a HyperCarb 2.1x150 mm from Thermo Scientific, Denmark, running at 25 degrees Celcius. Elution was performed with 5 mM AcNH4 aqueous solution and acetonitrile.

Ion chromatograms were extracted based on the common ionization patterns from the electrospray interfase. The peaks in the extracted chromatograms were evaluated based on the mass spectra and subsequently integrated, thus providing integrated responses for various carbohydrate species of the test sample.

**REFERENCES**

Buchholz (2005)  "Biocatalysts and Enzyme technology", Klaus Buchholz et al., ISBN-10: 3-527-30497-5, 2005, Wiley VCH Verlag GmbH

Franck (2002)  "Technological functionality of inulin and oligofructose", A. Franck, British Journal of Nutrition (2002), 87, Suppl. 2, S287-S291

Yun (1996)  "Fructooligosaccharides-Occurrence, preparation, and application", J. W. Yun, Enzyme and Microbial Technology 19: 107-117, 1996

Kunz (2000)  "Oligosaccharides in human milk: Structural, functional and metabolic aspects", Kunz et al., Ann. Rev. Nutr. 2000. 20:699-722

Simms *et al.* (1994)  Simms, P.J.; Hicks, K.B.; Haines, R.M.; Hotchkiss, A.T. and Osman, S.F.; (1994) Separations of lactose, lactobionic and lactobionolactose by high performance liquid chromatography. J. of Chromatography, 667, 67-73.

| Richmond *et al.* (1982) | Richmond, M.L.; Barfuss, D.L.; Harte, B.R.; Gray, J.I. and Stine, C.M.; (1982) Separation of Carbohydrates in Dairy Products by High Performance Liquid Chromatography, J. of Dairy Science, 65 (8), 1394-1400. |
| --- | --- |
| El Razzi (2002) | "Carbohydrate Analysis by Modern Chromatography and Electrophoresis", volume 66, Journal of Chromatography Library, Elsevier Science, 2002, ISBN-10: 0444500618 |
| Walstra *et al.* (2006) | "Dairy science and technology", Walstra et al., CRC Press, Second edition, 2006 |
| Scopes | Protein Purification: Principles and Practice; Robert K. Scopes; 3rd edition, Springer Verlag New York, Inc., ISBN 0-387-94072-3 |

WO 01/90,317 A2

EP 2 138 586 A1

WO 2009/113,030 A2

WO 2012/010,597 A1

**Claims**

1. A method of producing a composition comprising one or more galacto-oligosaccharide(s), the method comprising the steps of:

   a) providing a mixture comprising

      - a galactosyl donor comprising a galactosyl group bound to a leaving group, which galactosyl donor has a molar weight of at most 350 g/mol,
      - a galactosyl acceptor which is different from the galactosyl donor,
      said galactosyl acceptor is a saccharide or a sugar-alcohol, and

      wherein the molar ratio between the galactosyl acceptor and the galactosyl donor is at least 1:10, and wherein the mixture comprises at least 0.05 mol/L of the galactosyl acceptor,
      b) providing a first enzyme, said first enzyme having beta-galactosidase activity and transgalactosylating activity, said first enzyme contacting the mixture, and
      c) incubating the mixture and the first enzyme, thereby allowing the first enzyme to release the leaving group of the galactosyl donor and transfer the galactosyl group of the galactosyl donor to the galactosyl acceptor, thus forming the galacto-oligosaccharide, step c) furthermore comprising removing from the incubating mixture a leaving group released from the galactosyl donor and wherein the removal of the leaving group released from the galactosyl donor involves physical removal of free leaving groups from the incubating mixture and/or conversion of free leaving groups into one or more other chemical species which may still be present in the incubating mixture, thereby obtaining the composition comprising the one or more galacto-oligosaccharide(s).

2. The method according to claim 1, wherein the leaving group of the galactosyl donor is a glycosyl group.

3. The method according to claim 1 or 2, wherein the galactosyl donor is lactose or lactitol.

4. The method according to any of the preceding claims, furthermore comprising providing a microorganism which is capable of converting free leaving groups released from the galactosyl donor, and allowing said microorganism, during incubation, to remove a leaving group released from the galactosyl donor.

5. The method according to any of the preceding claims, furthermore comprising providing a second enzyme which is capable of converting free leaving groups released from the galactosyl donor, and allowing said second enzyme, during incubation, to convert a leaving group released from the galactosyl donor.

6. The method according to claim 5, wherein the second enzyme has glucose oxidase activity.

**7.** The method according to any of the claims 5-6, wherein

a) the specificity constant of the second enzyme relative to the free leaving group is at least 10 times higher than its specificity constant relative to the galactosyl acceptor,
b) the specificity constant of the second enzyme relative to the free leaving group is at least 10 times higher than its specificity constant relative to the galactosyl donor, or
c) the specificity constant of the second enzyme relative to the free leaving group is at least 10 times higher than its specificity constant relative to the mono-galactosylated galactosyl acceptor.

**8.** The method according to any of the claims 6-7, wherein the method furthermore comprises providing an enzyme having catalase activity which enzyme contacts the incubating mixture.

**9.** The method according to any of the claims 5-8, wherein the method furthermore comprises providing a removal agent capable of removing at least some of the conversion product obtained by converting the leaving group with the second enzyme, and allowing the removal agent, during the incubation, to remove at least some of the conversion product.

**10.** The method according to claim 9, wherein the removal agent comprises a salt of a divalent or trivalent metal ion.

**11.** The method according to claim 9, wherein the removal agent comprises, or even consists of, an anion exchange material.

**12.** The method according to any of the preceding claims, wherein step c) comprises addition of further galactosyl donor.

**13.** The method according to any of the preceding claims, wherein the concentration of galactosyl donor of the mixture during step c) is maintained at a concentration in the range of 0.01-1 mol/L.

**14.** The method according to any of the preceding claims furthermore comprising the step:

d) enriching the galacto-oligosaccharide of the composition of step c).


**Patentansprüche**

**1.** Verfahren zur Herstellung einer Zusammensetzung umfassend ein oder mehrere Galactooligosaccharide, wobei das Verfahren die folgenden Schritte umfasst:

a) Bereitstellen einer Mischung umfassend

- einen Galactosyldonor, welcher eine an eine Abgangsgruppe gebundene Galactosylgruppe umfasst, wobei der Galactosyldonor ein Molgewicht von höchstens 350 g/mol aufweist,
- einen Galactosylakzeptor, welcher sich von dem Galactosyldonor unterscheidet, wobei der Galactosylakzeptor ein Saccharid oder ein Zuckeralkohol ist, und

wobei das Molverhältnis zwischen dem Galactosylakzeptor und dem Galactosyldonor wenigstens 1:10 beträgt und wobei das Gemisch wenigstens 0,05 mol/l des Galactosylakzeptors umfasst,
b) Bereitstellen eines ersten Enzyms, wobei das erste Enzym eine beta-Galactosidaseaktivität und transgalaktosylierende Aktivität aufweist, wobei das erste Enzym die Mischung kontaktiert, und
c) Inkubieren des Gemisches und des ersten Enzyms, wodurch dem ersten Enzym ermöglicht wird, die Abgangsgruppe des Galactosyldonors freizusetzen und die Galactosylgruppe des Galactosyldonors auf den Galactosylakzeptor zu übertragen, wodurch das Galactooligosaccharid gebildet wird, wobei Schritt c) weiterhin ein Entfernen einer aus dem Galactosyldonor freigesetzten Abgangsgruppe aus der Inkubationsmischung umfasst und wobei das Entfernen der aus dem Galactosyldonor freigesetzten Abgangsgruppe ein physikalisches Entfernen von freien Abgangsgruppen aus der Inkubationsmischung und/oder eine Umwandlung der freien Abgangsgruppen in eine oder mehrere chemisches Spezies beinhaltet, welche noch in der Inkubationsmischung vorliegen können, wodurch die Zusammensetzung umfassend die ein oder mehreren Galactooligosaccharide erhalten wird.

**2.** Verfahren nach Anspruch 1, wobei die Abgangsgruppe des Galactosyldonors eine Glycosylgruppe ist.

**3.** Verfahren nach Anspruch 1 oder 2, wobei der Galactosyldonor Lactose oder Lactitol ist.

**4.** Verfahren nach einem der vorherigen Ansprüche, weiterhin umfassend Bereitstellen eines Mikroorganismus, welcher in der Lage ist, freie Abgangsgruppen umzuwandeln, welche aus dem Galactosyldonor freigesetzt werden, und Ermöglichen, dass der Mikroorganismus während der Inkubation eine Abgangsgruppe entfernt, welche aus dem Galactosyldonor freigesetzt wird.

**5.** Verfahren nach einem der vorherigen Ansprüche, weiterhin umfassend Bereitstellen eines zweiten Enzyms, welches in der Lage ist, freie Abgangsgruppen umzuwandeln, welche aus dem Galactosyldonor freigesetzt werden, und Ermöglichen, dass das zweite Enzym während der Inkubation eine Abgangsgruppe umwandelt, welche aus dem Galactosyldonor freigesetzt wird.

**6.** Verfahren nach Anspruch 5, wobei das zweite Enzym eine Glucoseoxidaseaktivität aufweist.

**7.** Verfahren nach einem der Ansprüche 5 - 6, wobei,

a) die Spezifitätskonstante des zweiten Enzyms relativ zur freien Abgangsgruppe wenigstens zehnmal höher als dessen Spezifitätskonstante relativ zum Galactosylakzeptor ist,
b) die Spezifitätskonstante des zweiten Enzyms relativ zur freien Abgangsgruppe wenigstens zehnmal höher als dessen Spezifitätskonstante relativ zum Galactosyldonor ist, oder
c) die Spezifitätskonstante des zweiten Enzyms relativ zur freien Abgangsgruppe wenigstens zehnmal höher als dessen Spezifitätskonstante relativ zum monogalactosylierten Galactosylakzeptor ist.

**8.** Verfahren nach einem der Ansprüche 6 - 7, wobei das Verfahren weiterhin ein Bereitstellen eines Enzyms mit Katalaseaktivität umfasst, wobei das Enzym die Inkubationsmischung kontaktiert.

**9.** Verfahren nach einem der Ansprüche 5 - 8, wobei das Verfahren weiterhin die Bereitstellung eines Entfernungsmittels umfasst, welches in der Lage ist, wenigstens einen Teil des Umwandlungsprodukts zu entfernen, welches durch die Umwandlung der Abgangsgruppe mit dem zweiten Enzym erhalten wird, und Ermöglichen, dass das Entfernungsmittel während der Inkubation wenigstens einen Teil des Umwandlungsprodukts entfernt.

**10.** Verfahren nach Anspruch 9, wobei das Entfernungsmittel ein Salz eines zweiwertigen oder dreiwertigen Metallions umfasst.

**11.** Verfahren nach Anspruch 9, wobei das Entfernungsmittel ein Anionenaustauschmaterial umfasst oder gar daraus besteht.

**12.** Verfahren nach einem der vorhergehenden Ansprüche, wobei Schritt c) die Zugabe von weiterem Galactosyldonor umfasst.

**13.** Verfahren nach einem der vorhergehenden Ansprüche, wobei die Konzentration von Galactosyldonor der Mischung während des Schrittes c) bei einer Konzentration im Bereich von 0,01 - 1 mol/l aufrechterhalten wird.

**14.** Verfahren nach einem der vorhergehenden Ansprüche, weiterhin umfassend den Schritt:

d) Anreicherung des Galactooligosaccharids der Zusammensetzung des Schrittes c).

**Revendications**

**1.** Procédé de production d'une composition comprenant un ou plusieurs galacto-oligosaccharides, le procédé comprenant les étapes consistant à :

a) fournir un mélange comprenant :

- un donneur de galactosyle comprenant un groupement galactosyle lié à un groupement quittant, lequel donneur de galactosyle a un poids moléculaire au maximum de 350 g/mole,
- un accepteur de galactosyle qui est différent du donneur de galactosyle, ledit accepteur de galactosyle étant un saccharide ou un alcool de sucre et

dans lequel le rapport molaire entre l'accepteur de galactosyle et le donneur de galactosyle est d'au moins 1:10 et dans lequel le mélange comprend au moins 0,05 mole/L de l'accepteur de galactosyle,
b) fournir un premier enzyme, ledit premier enzyme ayant une activité de bêta-galactosidase et une activité de transgalactosylation, ledit premier enzyme venant en contact avec le mélange, et
c) incuber le mélange et le premier enzyme, en permettant ainsi au premier enzyme de libérer le groupement quittant du donneur de galactosyle et de transférer le groupement galactosyle du donneur de galactosyle à l'accepteur de galactosyle, formant de la sorte le galacto-oligosaccharide, l'étape c) comprenant en outre l'élimination du mélange d'incubation d'un groupement quittant libéré par le donneur de galactosyle et dans lequel l'élimination du groupement quittant libéré du donneur de galactosyle implique l'élimination physique de groupements quittant libres du mélange d'incubation et/ou la conversion de groupements quittant libres en une ou plusieurs autres espèces chimiques qui peuvent encore être présentes dans le mélange d'incubation, en obtenant ainsi la composition comprenant les un ou plusieurs galacto-oligosaccharides.

2. Procédé selon la revendication 1, dans lequel le groupement quittant du donneur de galactosyle est un groupement glycosyle.

3. Procédé selon la revendication 1 ou 2, dans lequel le donneur de galactosyle est le lactose ou le lactitol.

4. Procédé selon l'une quelconque des revendications précédentes, comprenant en outre la fourniture d'un microorganisme qui est capable de convertir des groupements quittant libres libérés par le donneur de galactosyle et permettant audit microorganisme, au cours de l'incubation, d'éliminer un groupement quittant libéré par le donneur de galactosyle.

5. Procédé selon l'une quelconque des revendications précédentes, comprenant en outre la fourniture d'un second enzyme qui est capable de convertir des groupements quittant libres libérés par le donneur de galactosyle et permettant audit second enzyme, au cours de l'incubation, d'éliminer un groupement quittant libéré par le donneur de galactosyle.

6. Procédé selon la revendication 5, dans lequel le second enzyme a une activité d'oxydase de glucose.

7. Procédé selon l'une quelconque des revendications 5 - 6, dans lequel :

a) la constante de spécificité du second enzyme par rapport au groupement quittant libre est au moins 10 fois supérieure à sa constante de spécificité par rapport à l'accepteur de galactosyle,
b) la constante de spécificité du second enzyme par rapport au groupement quittant libre est au moins 10 fois supérieure à sa constante de spécificité par rapport au donneur de galactosyle, ou
c) la constante de spécificité du second enzyme par rapport au groupement quittant libre est au moins 10 fois supérieure à sa constante de spécificité par rapport à l'accepteur de galactosyle mono-galactosylé.

8. Procédé selon l'une quelconque des revendications 6 - 7, dans lequel le procédé comprend en outre la fourniture d'un enzyme ayant une activité de catalase, lequel enzyme vient en contact avec le mélange d'incubation.

9. Procédé selon l'une quelconque des revendications 5 - 8, dans lequel le procédé comprend en outre la fourniture d'un agent d'élimination capable d'éliminer au moins une certaine partie du produit de conversion obtenu en convertissant le groupement quittant avec le second enzyme et en permettant à l'agent d'élimination, au cours de l'incubation, d'éliminer au moins une certaine partie du produit de conversion.

10. Procédé selon la revendication 9, dans lequel l'agent d'élimination comprend un sel d'un ion métallique bivalent ou trivalent.

11. Procédé selon la revendication 9, dans lequel l'agent d'élimination comprend ou est même constitué d'un matériau d'échange anionique.

12. Procédé selon l'une quelconque des revendications précédentes, dans lequel l'étape c) comprend l'addition d'un autre donneur de galactosyle.

13. Procédé selon l'une quelconque des revendications précédentes, dans lequel la concentration de donneur de galactosyle du mélange au cours de l'étape c) est maintenue à une concentration dans la plage de 0,01 - 1 mole/L.

14. Procédé selon l'une quelconque des revendications précédentes, comprenant en outre l'étape consistant à :

d) enrichir le galacto-oligosaccharide de la composition de l'étape c).

# Fig. 1

Fig. 2

Free leaving groups

Allo-lactose and lactose

Oligosac -charides

Transgalactosylation by first enzyme

Donor

Acceptor

Free leaving group

## Fig. 3

Donor

Gal    Glc

Acceptor

Transgalactosylation
by first enzyme +
removal of leaving
groups

Free
leaving
groups

Oligosac-
charides

# Fig. 4

# Fig. 5

# Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0190317 A2
- WO 0190317 A
- EP 2138586 A1
- WO 2009113030 A2
- WO 2012010597 A1
- WO 2011120993 A1
- US 6555348 B2

### Non-patent literature cited in the description

- **J. SAMBROOK ; D.W. RUSSELL.** Molecular Cloning - A laboratory manual. 2001, 17.48-17.51
- *Dried milk, dried ice-mixes and processed cheese - Determination of lactose content - Part 2: Enzymatic method utilizing the galactose moiety of the lactose,* 2002
- **KLAUS BUCHHOLZ et al.** Biocatalysts and Enzyme technology. Wiley VCH Verlag GmbH, 2005, 3-527, 30497-5
- **A. FRANCK.** Technological functionality of inulin and oligofructose. *British Journal of Nutrition,* 2002, vol. 87 (2), S287-S291
- **J. W. YUN.** Fructooligosaccharides-Occurrence, preparation, and application. *Enzyme and Microbial Technology,* 1996, vol. 19, 107-117
- **KUNZ et al.** Oligosaccharides in human milk: Structural, functional and metabolic aspects. *Ann. Rev. Nutr.,* 2000, vol. 20, 699-722
- **SIMMS, P.J. ; HICKS, K.B. ; HAINES, R.M. ; HOTCHKISS, A.T. ; OSMAN, S.F.** Separations of lactose, lactobionic and lactobionolactose by high performance liquid chromatography. *J. of Chromatography,* 1994, vol. 667, 67-73
- **RICHMOND, M.L. ; BARFUSS, D.L. ; HARTE, B.R. ; GRAY, J.I. ; STINE, C.M.** Separation of Carbohydrates in Dairy Products by High Performance Liquid Chromatography. *J. of Dairy Science,* 1982, vol. 65 (8), 1394-1400
- Carbohydrate Analysis by Modern Chromatography and Electrophoresis. Journal of Chromatography Library. Elsevier Science, 2002, vol. 66
- **WALSTRA et al.** Dairy science and technology. CRC Press, 2006
- **ROBERT K. SCOPES.** Protein Purification: Principles and Practice. Springer Verlag New York, Inc,